# EUROPEAN PATENT SPECIFICATION

(11) **EP 4 186 413 B1**
(45) Date of publication and mention of the grant of the patent: **09.10.2024**
(21) Application number: 21857649.4
(22) Date of filing: 17.08.2021
(51) Int. Cl.: A61B 5/00

(54) **PHYSIOLOGICAL DATA ACQUISITION METHOD AND APPARATUS, AND WEARABLE DEVICE**
VERFAHREN UND VORRICHTUNG ZUR ERFASSUNG PHYSIOLOGISCHER DATEN UND WEARABLE-VORRICHTUNG
PROCÉDÉ ET APPAREIL D'ACQUISITION DE DONNÉES PHYSIOLOGIQUES ET DISPOSITIF À PORTER SUR SOI

(30) Priority: 18.08.2020 CN 202010834214
(43) Date of publication of application: 31.05.2023
(73) Proprietor: Huawei Technologies Co., Ltd., Shenzhen, Guangdong 518129 (CN)
(72) Inventor: LI, Jing, Shenzhen, Guangdong 518129 (CN); ZHANG, Yue, Shenzhen, Guangdong 518129 (CN)
(74) Representative: Goddar, Heinz J.
(86) International application number: PCT/CN2021/112892
(87) International publication number: WO 2022/037555

(56) References cited:
- EP-A1- 3 671 757
- WO-A1-2018/098719
- CN-A- 104 375 623
- CN-A- 104 765 269
- CN-A- 104 939 817
- CN-A- 105 434 043
- JP-A- 2004 254 827
- US-A1- 2016 374 569
- US-A1- 2019 282 159

## Description

This application claims priority to Chinese Patent Application No. 202010834214.4, filed with the China National Intellectual Property Administration on August 18, 2020 and entitled "PHYSIOLOGICAL DATA COLLECTION METHOD AND APPARATUS, AND WEARABLE DEVICE.

### TECHNICAL FIELD

This application relates to the field of data collection technologies, and in particular, to a physiological data collection method and apparatus, and a wearable device.

### BACKGROUND

Sleep is one of the most important physiological activities of human beings. Research shows that a large quantity of people have a sleep trouble and even an insomnia problem. Therefore, it is critical to accurately perform sleep monitoring to help users better understand their sleep status. To implement sleep monitoring, in a current technology, sensors that can collect physiological data are disposed in wearable devices such as bands or watches, and these sensors are used to collect physiological data of users. Then, analysis is performed based on the collected physiological data, to implement sleep monitoring for the users.

Sleep can be classified into long-time sleep and short-time sleep. The long-time sleep, for example, nighttime sleep, means that sleep duration reaches a specific duration threshold. The short-time sleep, a noontime snooze and a nap for example, means that sleep duration is short and does not reach a specific duration threshold. The long-time sleep is a main way that the users rest, and is also a sleep type that attracts most user attention. With long sleep duration, the long-time sleep provides the wearable devices with access to detection of a large amount of data from the users in sleep, and it is possible to perform refined sleep analysis. Refined sleep analysis is performed based on the long-term sleep, and sleep quality scores and suggestions are provided. This helps the users learn about a sleep status and make targeted improvements. US 2019/0282159 A1 describes an automated detection of breathing disturbances; a system receives user state data generated over a time period by a combination of sensors provided via a wearable tracker associated with the user and the system can use this information to calculate a sleep fitness score, breathing disturbance score, or other such value.

To implement long-time sleep monitoring, in the current technology, related sensors in the wearable devices are continuously enabled, and physiological data of the users is continuously collected. Then, physiological data during the long-time sleep is identified from the collected physiological data and analyzed. In this way, physiological data collection during the long-time sleep of the users can be implemented, and sleep monitoring for the users can be implemented. However, both physiological data collection and sleep monitoring require high power consumption. As a result, the wearable devices consume electricity soon with a short battery life, and user experience is poor.

### SUMMARY

In view of this, embodiments of this application provide a physiological data collection method and apparatus, and a wearable device, to resolve a problem, in a current technology, of high power consumption for collecting physiological data of a user during long-time sleep.

A first aspect of embodiments of this application provides a physiological data collection method, applied to a wearable device including a first sensor, and the method includes:
detecting whether a user falls asleep;
identifying, when it is detected that the user falls asleep, whether a first quantity of times that the user enters long-time sleep within a first time period is less than a quantity-of-time threshold;
detecting whether the user wakes up;
when a threshold of the first quantity of times is less than the quantity-of-time threshold, enabling a first sensor, and collecting first physiological data of the user by using the first sensor; and
when it is detected that the user wakes up, disabling the first sensor.

In this embodiment of this application, in a period in which sleep monitoring keeps enabled, the sensor is not continuously enabled, but falling-asleep monitoring is continuously performed on the user. When it is detected that the user falls asleep, it is determined whether a quantity of times that the user is in long-time sleep in a recent first time period reaches the quantity-of-time threshold. In addition, the sensor is enabled only when the quantity-of-time threshold is not reached, to collect physiological data of the user. In addition, when the user is in sleep, waking-up monitoring continues to be performed on the user, and the sensor is disabled when it is detected that the user wakes up. The first time period is a past time period, and a specific time period range may be set by a person skilled in the art based on an actual situation.

In normal life and work, the user is in a sober state most of the time. Therefore, compared with keeping a related sensor enabled all day, this embodiment of this application can reduce a large amount of sensor collection work and power consumption. In addition, setting a quantity-of-time threshold is set can effectively avoid a case in which physiological data collection is performed on all sleep periods of the user, and makes it less probable to enable a related sensor for short-time sleep. Therefore, in this embodiment of this application, a workload of collecting physiological data during long-time sleep by the sensor can be reduced as well as power consumption, for lower electricity consumption and a longer battery life of the wearable device.

In a first possible implementation of the first aspect, the identifying, when it is detected that the user falls asleep, whether a first quantity of times that the user enters long-time sleep within a first time period is less than a quantity-of-time threshold includes:
identifying, when it is detected that the user falls asleep, whether a falling-asleep moment is within a second time period; and
when the falling-asleep moment is within the second time period, identifying whether the first quantity of times that the user enters long-time sleep within the first time period is less than the quantity-of-time threshold.

The wearable device can obtain historical sleep data of long-time sleep of the user. In this case, in this embodiment of this application, a falling-asleep moment range (namely, the second time period) of an actual long-time sleep period of the user is estimated in advance based on a long-time sleep habit of the user. In a period in which sleep monitoring keeps enabled, the sensor is not continuously enabled, but falling-asleep monitoring is continuously performed on the user. When it is detected that the user falls asleep, it is determined whether a falling-asleep moment is within the falling-asleep moment range. When the falling-asleep moment is within the falling-asleep moment range, it indicates that the sleep is of a great possibility to be long-time sleep. Therefore, it is determined whether a quantity of times that the user is in long-time sleep in the first time period reaches the quantity-of-time threshold.

It is considered that in actual life, long-time sleep is an extremely regular behavior for the user. Therefore, in this embodiment of this application, historical sleep data of the user is analyzed to adaptively obtain a falling-asleep moment range that the user is accustomed to, so that whether the sleep of the user may be long-time sleep can be accurately distinguished. Therefore, in this embodiment of this application, whether to enable the sensor is determined based on three conditions: whether the user falls asleep, whether a falling-asleep moment is within the falling-asleep moment range that the user is accustomed to, and whether a quantity of long-time sleep times within a recent time period reaches the quantity-of-time threshold. The intent of the user each time the user falls asleep can be accurately identified, to implement accurate identification for long-time sleep. Therefore, a case in which physiological data collection is performed on all sleep periods of the user can be effectively avoided, and a probability of enabling a related sensor for short-time sleep is reduced. Power consumption for physiological data collection can be reduced.

Based on the first possible implementation of the first aspect, in a second possible implementation of the first aspect, before the identifying whether a falling-asleep moment is within a second time period, the method further includes:
obtaining historical sleep data of the user within a third time period; and
analyzing the historical sleep data, to obtain the second time period.

In this embodiment of this application, historical sleep data of the user within a past time period (that is, the third time period) is obtained. Based on the obtained historical sleep data, in this embodiment of this application, the historical sleep data is analyzed, to obtain a falling-asleep moment range in which the user enters long-time sleep on a daily basis. In this way, adaptive learning for a falling-asleep habit of a user is implemented, so that the falling-asleep moment range is more applicable to the user. Therefore, falling-asleep detection for long-time sleep is more accurate and reliable.

Based on the second possible implementation of the first aspect, in a third possible implementation of the first aspect, the analyzing the historical sleep data, to obtain the second time period includes:
analyzing the historical sleep data, to obtain sleep period data that is associated with k times of sleep in which the user sleeps for the longest sleep duration each day and that are within the third time period, where k is equal to the quantity-of-time threshold;
picking out, from the sleep period data, sleep period data that is associated with a sleep period whose sleep duration is greater than or equal to a duration threshold, and reading a falling-asleep moment included in each piece of picked-out sleep period data; and
analyzing the read falling-asleep moment, to obtain the second time period.

In this embodiment of this application, a sleep status each day within the third time period is respectively analyzed, to obtain sleep period data whose quantity is a threshold of a quantity of times of the longest sleep duration each day. Then, picking is performed by using a duration threshold, to obtain sleep period data during actual long-time sleep each day within the third time period. Finally, a falling-asleep moment is extracted from the sleep period data, and the falling-asleep moment is analyzed, to obtain the second time period. Therefore, in this embodiment of this application, adaptive learning and accurate extraction for a moment at which a user enters long-time sleep each day can be implemented, so that falling-asleep detection for long-time sleep is more accurate and reliable.

Based on the third possible implementation of the first aspect, in a fourth possible implementation of the first aspect, after the sleep period data is picked out, the method further includes:
obtaining sleep duration corresponding to the picked-out sleep period data, and updating the duration threshold based on the sleep duration.

The duration threshold is updated by using the historical sleep data, so that a value of the duration threshold can be adaptive to an actual sleep habit of the user. Therefore, in a process of identifying long-time sleep of the user, accuracy of identifying long-time sleep of the user can be higher, so that a more accurate occasion at which the sensor is enabled is selected, and possibilities of enabling the sensor by mistakes are reduced. Therefore, power consumption for collecting physiological data of the user during long-time sleep can be reduced.

Based on the first to the fourth possible implementations of the first aspect, in a fifth possible implementation of the first aspect, the method further includes:
when it is detected that the user wakes up, collecting statistics on first duration lasting from the moment at which the user falls asleep to the moment at which the user wakes up;
when the first duration is greater than or equal to the duration threshold, determining that the sleep is long-time sleep, and updating the first quantity of times.

In this embodiment of this application, after it is determined that the sleep is long-time sleep, the first quantity of times is updated timely, to ensure accuracy of the first quantity of times.

Based on the fifth possible implementation of the first aspect, in a sixth possible implementation of the first aspect, the method further includes:
when the first duration is greater than or equal to the duration threshold, analyzing first physiological data collected this time, to obtain corresponding sleep analysis data, so that monitoring on the long-time sleep is implemented.

Based on the first to the fourth possible implementations of the first aspect, in a seventh possible implementation of the first aspect, the method further includes:
when it is detected that the user wakes up, collecting statistics on first duration lasting from the moment at which the user falls asleep to the moment at which the user wakes up;
when the first duration is less than the duration threshold, detecting whether the user falls asleep;
when it is detected that the user falls asleep, detecting whether the user wakes up, enabling the first sensor, and collecting second physiological data of the user by using the first sensor; and
when it is detected that the user wakes up, disabling the first sensor.

In this embodiment of this application, to prevent a case in which a user needs to enter long-time sleep again because normal long-time sleep of the user is interrupted cannot be identified, when it is identified that the sleep duration of the user is less than the duration threshold, the sensor is set to be enabled when the user performs a next falling-asleep behavior. To be specific, in this case, determining whether the user is in the falling-asleep moment range and whether the quantity-of-time threshold is reached is no longer performed, but the sensor is directly enabled when it is detected that the user falls asleep, and physiological data of the user is collected. According to this embodiment of this application, even if the user is interrupted by an interference factor such as noise during long-time sleep, physiological data collection can be performed on the user timely when the user re-enters long-time sleep. In this way, physiological data collection performed during long-time sleep is more accurate and reliable.

Based on the third or the fourth possible implementation of the first aspect, in an eighth possible implementation of the first aspect, the obtaining historical sleep data of the user within a third time period includes:
identifying a time period type of a current time period, to obtain a first type;
picking out, from the third time period, a fourth time period whose time period type is the first type; and
obtaining the historical sleep data within the fourth time period.

In this embodiment of this application, when a falling-asleep time period is analyzed, a time period (that is, the fourth time period) of a time period type to which the current time period belongs is selected from a past time period, and historical sleep data in these time periods is obtained in a targeted manner. Therefore, in this embodiment of this application, a falling-asleep moment range can be analyzed based on the time period type to which the current time period belongs, to implement accurate learning and identification for the sleep habit of the user. In this way, in this embodiment of this application, identification for long-time sleep of the user is more accurate, and the sensor is enabled at a more accurate and effective occasion.

Based on the third or the fourth possible implementation of the first aspect, in a ninth possible implementation of the first aspect, the analyzing the historical sleep data, to obtain the second time period includes:
dividing the third time period into a plurality of time period sets, and extracting sleep sub-data of each time period set from the historical sleep data, where each time period set includes only time periods of a same time period type, and different time period sets are associated with different time period types; and
respectively analyzing the sleep sub-data associated with each time period set, to obtain the second time period respectively associated with each time period type; and
correspondingly, the identifying, when it is detected that the user falls asleep, whether a falling-asleep moment is within a second time period includes:
   when it is detected that the user falls asleep, identifying a time period type of a current time period, to obtain a second type; and
   obtaining the second time period associated with the second type, and identifying whether the falling-asleep moment is within the second time period.

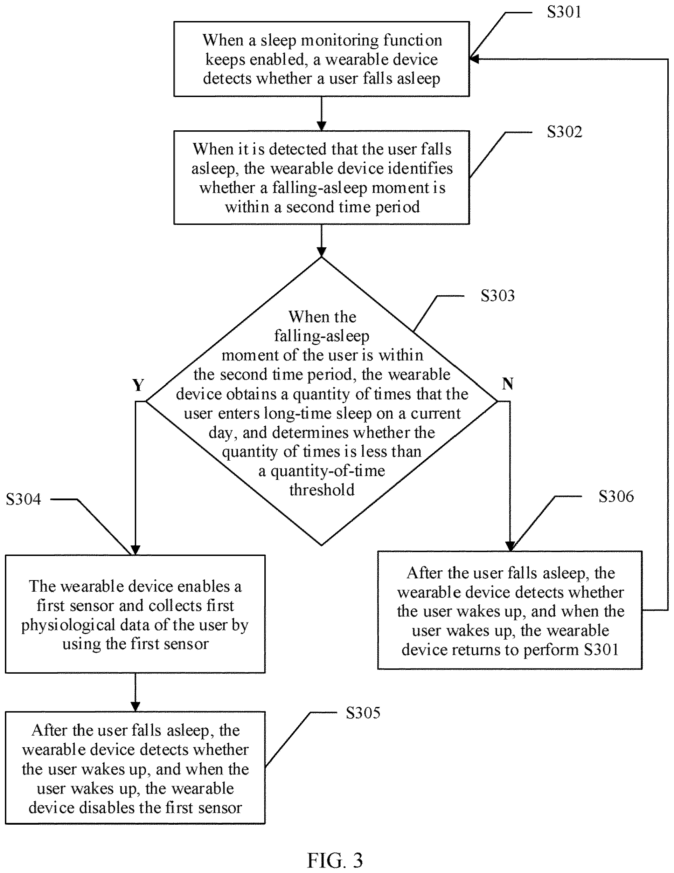

In this embodiment of this application, each time physiological data of the user during long-time sleep is successfully obtained and the historical sleep data is updated, a falling-asleep habit of the user in each time period type is analyzed timely, to obtain a falling-asleep moment range of the user in each time period type and implement adaptive learning for the falling-asleep habits of the user. However, in the embodiment shown in FIG. 3, after it is detected that the user falls asleep, in S302, a falling-asleep moment can be determined only by reading the falling-asleep moment range of the time period type to which the analyzed current time period belongs. Therefore, in this embodiment of this application, adaptive and accurate learning for a sleep habit of the user can be implemented. In this way, in this embodiment of this application, identification for long-time sleep of the user is more accurate, and the sensor is enabled at a more accurate and effective occasion.

In a tenth possible implementation of the first aspect, the identifying, when it is detected that the user falls asleep, whether a first quantity of times that the user enters long-time sleep within a first time period is less than a quantity-of-time threshold includes:
when it is detected that the user falls asleep, identifying whether the falling asleep is the first time that the user falls asleep within the first time period; and
when the falling asleep is the first time that the user falls asleep within the first time period, determining that the threshold of the first quantity of times is less than the quantity-of-time threshold.

In this embodiment of this application, when it is detected that the user falls asleep, it is identified whether the falling asleep is the first time that the user falls asleep within the first time period. If the falling asleep is the first time that the user falls asleep, a specific value of a quantity of times that the user enters long-time sleep may not be obtained (that is, regardless of the value of the first quantity of times); instead, it is determined that the quantity of times is less than the quantity-of-time threshold, and physiological data collection in a next step is started.

A second aspect of embodiments of this application provides a physiological data collection apparatus, including:
a falling-asleep detection module, configured to detect whether a user falls asleep;
a falling-asleep quantity-of-time detection module, configured to: when it is detected that the user falls asleep, identify whether a first quantity of times that the user enters long-time sleep within a first time period is less than a quantity-of-time threshold;
a waking-up detection module, configured to detect whether the user wakes up;
a data collection module, configured to: when a threshold of the first quantity of times is less than the quantity-of-time threshold, enable a first sensor, and collect first physiological data of the user by using the first sensor; and
a sensor disabling module, configured to: when it is detected that the user wakes up, disable the first sensor.

In a first possible implementation of the second aspect, the falling-asleep quantity-of-time detection module includes:
a falling-asleep moment identification module, configured to: when it is detected that the user falls asleep, identify whether a falling-asleep moment is within a second time period; and
a quantity-of-time detection module, configured to: when the falling-asleep moment is within the second time period, identify whether the first quantity of times that the user enters long-time sleep within the first time period is less than the quantity-of-time threshold.

Based on the first possible implementation of the second aspect, in a second possible implementation of the second aspect, the physiological data collection apparatus further includes:
a historical data obtaining module, configured to obtain historical sleep data of the user within a third time period; and
a historical data analysis module, configured to analyze the historical sleep data, to obtain the second time period.

Based on the second possible implementation of the second aspect, in a third possible implementation of the second aspect, the historical data analysis module includes:
a sleep analysis module, configured to analyze the historical sleep data, to obtain sleep period data that is associated with k times of sleep in which the user sleeps for the longest sleep duration each day and that are within the third time period, where k is equal to the quantity-of-time threshold;
a sleep picking module, configured to: pick out, from the sleep period data, sleep period data that is associated with a sleep period whose sleep duration is greater than or equal to a duration threshold, and read falling-asleep moment included in each piece of picked-out sleep period data; and
a time analysis module, configured to analyze the read falling-asleep moment, to obtain the second time period.

Based on the third possible implementation of the second aspect, in a fourth possible implementation of the second aspect, the physiological data collection apparatus further includes:
a duration threshold updating module, configured to: obtain sleep duration corresponding to the picked-out sleep period data, and update the duration threshold based on the sleep duration.

Based on the first to the fourth possible implementations of the second aspect, in a fifth possible implementation of the second aspect, the apparatus further includes:
a duration statistics collection module, configured to: when it is detected that the user wakes up, collect statistics on first duration lasting from the moment at which the user falls asleep to the moment at which the user wakes up;
a quantity-of-time updating module, configured to: when the first duration is greater than or equal to the duration threshold, determine that the sleep is long-time sleep, and update the first quantity of times.

Based on the fifth possible implementation of the second aspect, in a sixth possible implementation of the second aspect, the apparatus further includes:
a sleep analysis module, configured to: when the first duration is greater than or equal to the duration threshold, analyze first physiological data collected this time, to obtain corresponding sleep analysis data, so that monitoring for the long-time sleep is implemented.

Based on the first to the fourth possible implementations of the second aspect, in a seventh possible implementation of the second aspect, the physiological data collection apparatus further includes:
a duration statistics collection module, configured to: when it is detected that the user wakes up, collect statistics on first duration lasting from the moment at which the user falls asleep to the moment at which the user wakes up;
a falling-asleep detection module, configured to: when the first duration is less than the duration threshold, detect whether the user falls asleep;
a secondary data collection module, configured to: when it is detected that the user falls asleep, enable a first sensor, and collect second physiological data of the user by using the first sensor;
a waking-up detection module, configured to detect whether the user wakes up; and
a sensor disabling module, configured to: when it is detected that the user wakes up, disable the first sensor.

Based on the third or the fourth possible implementation of the second aspect, in an eighth possible implementation of the second aspect, the historical data obtaining module includes:
a first type identification module, configured to identify a time period type of a current time period, to obtain a first type;
a time period picking module, configured to pick out, from the third time period, a fourth time period whose time period type is the first type; and
a data obtaining module, configured to obtain historical sleep data within the fourth time period.

Based on the third or the fourth possible implementation of the second aspect, in a ninth possible implementation of the second aspect, the historical data analysis module includes:
a data division module, configured to: divide the third time period into a plurality of time period sets, and extract sleep sub-data of each time period set from the historical sleep data, where each time period set includes only time periods of a same time period type, and different time period sets are associated with different time period types; and
a data analysis module, configured to respectively analyze the sleep sub-data associated with each time period set, to obtain the second time period respectively associated with each time period type.

Correspondingly, the falling-asleep moment identification module includes:
a second type identification module, configured to: when it is detected that the use falls asleep, identify a time period type of a current time period, to obtain a second type;
a time identification module, configured to: obtain the second time period associated with the second type, and identify whether the falling-asleep moment is within the second time period.

In a tenth possible implementation of the second aspect, the falling-asleep quantity-of-time detection module includes:
a first-time falling-asleep identification module, configured to: when it is detected that the user falls asleep, identify whether the sleep is the first time that the user falls asleep within the first time period; and
a determining module, configured to: when the sleep is the first time that the user falls asleep within the first time period, determine that the threshold of the first quantity of times is less than the quantity-of-time threshold.

A third aspect of embodiments of this application provides a wearable device. The wearable device includes a memory and a processor. The memory stores a computer program that can run on the processor. When the processor executes the computer program, the wearable device is enabled to implement the steps of any physiological data collection method in the first aspect.

A fourth aspect of embodiments of this application provides a computer-readable storage medium. The computer-readable storage medium stores a computer program. When the computer program is executed by a processor, the wearable device is enabled to implement the steps of any physiological data collection method in the first aspect.

A fifth aspect of embodiments of this application provides a computer program product. When the computer program product runs on a wearable device, the wearable device is enabled to perform the physiological data collection method according to any one of the first aspect.

A sixth aspect of embodiments of this application provides a chip system. The chip system includes a processor. The processor is coupled to a memory. The processor executes a computer program stored in the memory, to implement the physiological data collection method according to any one of the first aspect.

The chip system may be chip module including a single chip or a plurality of chips.

It may be understood that, for beneficial effects of the second aspect to the sixth aspect, refer to related descriptions in the first aspect. Details are not described herein again.

### BRIEF DESCRIPTION OF DRAWINGS

FIG. 1 is a schematic flowchart of a physiological data collection method according to an embodiment of this application;
FIG. 2 is a schematic flowchart of a physiological data collection method according to an embodiment of this application;
FIG. 3 is a schematic flowchart of a physiological data collection method according to an embodiment of this application;
FIG. 4 is a schematic flowchart of a physiological data collection method according to an embodiment of this application;
FIG. 5 is a schematic flowchart of a physiological data collection method according to an embodiment of this application;
FIG. 6 is a schematic flowchart of a physiological data collection method according to an embodiment of this application;
FIG. 7A and FIG. 7B are a schematic flowchart of a physiological data collection method according to an embodiment of this application;
FIG. 8 is a schematic flowchart of a physiological data collection method according to an embodiment of this application;
FIG. 9 is a schematic flowchart of a physiological data collection method according to an embodiment of this application;
FIG. 10 is a schematic diagram of a structure of a physiological data collection apparatus according to an embodiment of this application; and
FIG. 11 is a schematic diagram of a structure of a wearable device according to an embodiment of this application.

### DESCRIPTION OF EMBODIMENTS

In the following description, to illustrate rather than limit, specific details such as a particular system structure and technology are provided, to facilitate thorough understanding of embodiments of this application. However, a person skilled in the art should know that this application may also be implemented in other embodiments without these specific details. In other cases, detailed descriptions of well-known systems, apparatuses, circuits, and methods are omitted, so that this application is described without being obscured by unnecessary details.

For ease of understanding this application, embodiments of this application are first briefly described herein.

Long-time sleep and short-time sleep are two sleep types classified based on sleep duration. The long-time sleep means that sleep duration reaches a specific duration threshold, for example, sleep at night. The short-time sleep means that sleep duration is short and does not reach a specific duration threshold, for example, an afternoon nap and a nap.

To monitor long-time sleep of a user, physiological data of the user during the long-time sleep needs to be collected first. To implement physiological data collection during long-time sleep, there are the following several optional practices in a related technology.

1. Sensors related to collection of physiological data are turned on around the clock, to continuously collect physiological data.

In this case, as long as the user performs long-time sleep, the collected physiological data includes physiological data during the long-time sleep. Therefore, integrity of physiological data collected during long-time sleep is high. However, collection needs to be performed for a long time period. Therefore, power consumption of the sensor is high, electricity consumption of the wearable device is fast, and battery life is poor. In addition, excessive physiological data is collected. Therefore, great load is brought to data storage and data processing of the wearable device, and costs of the wearable device is increased.

2. A time period for collecting physiological data is preset by a skilled person, for example, from 22:00 to 10:00. The wearable device fixedly keeps a related sensor enabled within the time period every day, to collect physiological data. Outside the time period, the related sensor is disabled by default.

In this case, the wearable device may obtain physiological data in a fixed time period of each day. In one aspect, for some users who are accustomed to performing long-time sleep within the time period, complete physiological data during the long-time sleep may be collected in this case. However, for a user accustomed to performing long-time sleep within another time period, in this case, it is difficult to collect complete physiological data during the long-time sleep, and even the physiological data during the long-time sleep may not be fully collected. For example, some users on a night shift usually perform long-time sleep in the daytime. In this case, complete physiological data of the user cannot be collected during long-time sleep within a time period lasting from 22:00 to 10:00. In another aspect, a set time period usually can hardly fully match with an actual long-time sleep time period of the user. Therefore, some physiological data that is not within the long-time sleep period is collected, and the collected physiological data cannot be used for sleep monitoring. Therefore, the sensor performs an unwanted operation, and power consumption of the wearable device is increased.

To effectively collect physiological data of the user during long-time sleep, and reduce power consumption of the wearable device caused by physiological data collection, in embodiments of this application, whether the user falls asleep is first detected. A logic for determining whether to enable the sensor is triggered only when the user falls asleep. In addition, it is considered that a quantity of times that the user performs long-time sleep within a specific time period in actual life is very limited. For example, in a day, usually only one long-time sleep is performed. However, in addition to long-time sleep, the user may also perform short-time sleep within a period of time. Therefore, when the sensor is enabled each time the user falls asleep, the sensor inevitably collects excessive useless physiological data. This results in an increase in power consumption. Therefore, in embodiments of this application, a quantity-of-time threshold of long-time sleep is set based on an actual situation of the user. When it is detected that the user falls asleep, it is determined whether a quantity of times that the user performs long-time sleep in a recent period of time reaches the quantity-of-time threshold. When the quantity-of time threshold is not reached, it indicates that the sleep may be long-time sleep. In this case, in embodiments of this application, the sensor is enabled to collect physiological data of the user. When it is detected that the user wakes up, that is, after the sleep ends, in embodiments of this application, the sensor is disabled, to end the physiological data collection.

In embodiments of this application, the sensor is enabled when the user falls asleep and a quantity of times that the user performs long-time sleep in a recent period of time does not reach the quantity-of-time threshold. Compared with that in a related technology, a case in which the sensor keeps enabled in a time period in which the user is awake is avoided. In normal life and work, the user is in a sober state most of the time. Therefore, compared with keeping a related sensor enabled all day, this embodiment of this application can reduce a large amount of sensor collection work and power consumption. Compared with fixedly keeping a sensor enabled in a preset time period, this can better adapt to an actual sleep habit of the user, and reduces cases in which physiological data of the user in an awake state is collected. In addition, setting the quantity-of-time threshold can effectively avoid a case in which physiological data collection is performed on all sleep periods of the user, and makes it less probable to enable a related sensor for short-time sleep. Therefore, power consumption for physiological data collection can be reduced. In conclusion, in embodiments of this application, physiological data collection performed when the user is awake can be avoided, and cases in which physiological data collection is performed when the user is in short-time sleep can be reduced. Physiological data can be accurately collected during long-time sleep. Therefore, a workload of collecting physiological data during long-time sleep by a related sensor can be reduced as well as power consumption, for lower electricity consumption and a longer battery life of the wearable device.

In addition, some terms that may be used in embodiments of this application are described as follows.

Duration threshold: Long-time sleep is an important way for a user to rest to restore physical functions and mentality. Therefore, long-time sleep needs to have long duration, to ensure that the user rests sufficiently. In embodiments of this application, the duration threshold is used to measure whether single sleep duration of the user is sufficient. In other words, long-time sleep and short-time sleep are distinguished by using the duration threshold. When the sleep duration reaches the duration threshold, it may be considered that the sleep is long-time sleep. On the contrary, when the duration threshold is not reached, it may be considered that the sleep is short-time sleep. In actual application, the duration threshold may be set by a skilled person. For example, the duration threshold may be set to any duration within 5 hours to 6 hours. Alternatively, the duration threshold may be determined after a skilled person surveys and collects statistics on sleep duration of the user. Alternatively, analysis may be performed based on an actual sleep habit of the user, to obtain a duration threshold appropriate to the user. This is not specifically and excessively limited herein.

Sleep monitoring: In embodiments of this application, sleep monitoring refers to monitoring long-time sleep of a user. Specifically, physiological data collected during long-time sleep of the user is analyzed, and a specific analysis result (that is, sleep analysis data) is generated, to help the user learn a long-time sleep status of the user. The analysis result may be sleep data parsing (for example, duration of three sleep periodicities: deep sleep, light sleep, and rapid eye movement sleep, and distribution time of each periodicity during the entire long-time sleep), a sleep quality score, or a sleep guidance suggestion, or may be other content. This may be specifically set by a skilled person based on an actual requirement. Physiological data (including first physiological data and second physiological data) in the following embodiments of this application is physiological data used for performing sleep monitoring on the user. A specific type of physiological data collected during sleep monitoring and an analysis method for sleep monitoring are not excessively limited in embodiments of this application. For example, any one or more of a heart rate, a pulse waveform, and bioimpedance may be used as the physiological data in embodiments of this application. Based on different selected physiological data, an electrocardiogram (electrocardiogram, ECG), a photo plethysmo graphy (Photo Plethysmo Graphy, PPG) method, or a bioelectrical impedance analysis (Bioelectrical Impedance Analysis, BIA) method may be used as a corresponding analysis method in embodiments of this application.

It should be noted that, in embodiments of this application, sleep monitoring is a function provided by the wearable device. In actual application, the sleep monitoring function may be set to be always enabled by default, or an option of enabling or disabling the function may be provided for the user. This may be specifically set by a manufacturer of the wearable device. When the sleep monitoring function is set to be always enabled by default, the physiological data collection method provided in embodiments of this application is performed when the user uses the wearable device. When the option of enabling or disabling the sleep monitoring function is provided for the user, the physiological data collection method provided in embodiments of this application is performed when the user chooses to enable the sleep monitoring function. Therefore, all the following embodiments of this application are performed when the sleep monitoring function keeps enabled.

A first sensor refers to a sensor that collects physiological data used for performing sleep monitoring on the user. In embodiments of this application, unless otherwise specified, a "sensor" refers to the "first sensor". Therefore, a type of the first sensor needs to be determined based on physiological data that actually needs to be collected. This is not excessively limited herein. For example, when the physiological data that needs to be collected is a heart rate, a heart rate sensor may be used as the first sensor in embodiments of this application. When the physiological data that needs to be collected is a pulse waveform, an optical heart rate sensor may be used as the first sensor. When the physiological data that needs to be collected is bioimpedance, a bioelectrical impedance sensor may be used as the first sensor. In addition, a quantity of first sensors is not excessively limited in embodiments of this application, and may be set by a skilled person based on an actual requirement.

Historical sleep data refers to sleep-related data of the user within a past period of time (that is, a third time period). Data content specifically included in the historical sleep data may be set by a skilled person based on an actual requirement. This is not limited herein. For example, the historical sleep data may be set to include a falling-asleep moment and a waking-up moment in long-time sleep within a past period of time. Alternatively, the historical sleep data may be set to include falling-asleep moments and waking-up moments of all sleep periods within a past period of time. Alternatively, the historical sleep data may be set to include falling-asleep moments and waking-up moments of all sleep periods within a past period of time, and further include physiological data of the user collected during long-time sleep. In addition, a specific range of the past period of time is not limited herein, and may be set by a skilled person as required. For example, the range may be set to a past week or a past month.

User activity day refers to the following: In actual life, calendar days are usually divided from 00:00 of each day to 00:00 of a next day (that is, from 00:00 of a current day to 00:00 of a next day; herein, to facilitate description of a time period range of each calendar day, a minimum time scale is set to a second; theoretically, the minimum time scale may be further refined, for example, set to a millisecond; and this is not limited herein). In other words, a time period lasting from 00:00 to 24:00 each day is considered as a time period of a current day. However, in an actual situation, a user performs long-time sleep mostly across calendar days. For example, night sleep usually lasts from night of a current day to morning of a next day, and crosses 00:00 of the next day. Therefore, when physiological data collection and analysis are performed on the user during long-time sleep each day, when data is distinguished in a traditional calendar day manner, physiological data may be improperly separated.

To better adapt to occurrence time characteristics of long-time sleep, and implement proper physiological data collection and analysis during long-time sleep, a concept of "user activity day" is proposed in embodiments of this application. The user activity day refers to a time period lasting from a start moment on a calendar day to an end moment 24 hours later. The start moment may be any moment from 00:00 on the calendar day to 24:00 on the calendar day, and the end moment is a moment 24 hours later than the start moment (that is, duration of the user activity day is also 24 hours). For example, if the start moment of the user activity day is 20:00:00, the end moment is 19:59:59 on a next calendar day (that is, from 8:00 p.m. on the calendar day to a moment before 8:00 p.m. on the next calendar day; herein, to facilitate description of a time period range of each user activity day, a minimum time scale is set to a second; theoretically, the minimum time scale may be further refined, for example, set to a millisecond; and this is not limited herein). In this case, a period lasting from 20:00:00 on the current calendar day to 19:59:59 on the next calendar day is referred to as a user activity day. The start moment of the user activity day is not excessively limited in embodiments of this application, and may be set by a skilled person skilled or a user based on an actual requirement. For example, for a user who performs long-time sleep at night, the start moment may be set to a time point in the afternoon of a calendar day, for example, may be set to 8:00. For some users who need to perform long-time sleep in the daytime (for example, some users who need to be on duty at night), the start moment may be set to a time point in the early morning, for example, 3:00. When the start moment is set to 00:00, the user activity day is the same as the calendar day. In other words, the user activity day is the calendar day.

It should be specially noted that, in the following embodiments of this application, unless otherwise specified in the embodiments, a "current day" in the embodiments refers to a current user activity day rather than a current calendar day by default. "Each day" refers to each user activity day.

Quantity-of-time threshold: Generally, a quantity of times that a user performs long-time sleep each day is limited. In one aspect, duration of a day is limited, and long-time sleep takes a long time period each time. Therefore, it is difficult to perform long-time sleep for a plurality of times in a day. In another aspect, as a main way of rest to restore physical function and spirit, in a normal case, long-time sleep for a small quantity of times can achieve a rest effect. Long-time sleep for a large quantity of times causes a human body to fall into a faint state, which deteriorates the rest effect. Based on this actual situation, a quantity-of-time threshold is set in embodiments of this application, to measure whether the user performs long-time sleep each day for a large quantity of times. When the quantity of times is less than the quantity-of-time threshold, it is considered that the quantity of times is small, and subsequent sleep may still be long-time sleep. When the quantity of times is greater than or equal to the quantity-of-time threshold, it is considered that the quantity of times is relatively large, and there is a high probability that subsequent sleep of the user is not long-time sleep. When the quantity-of-time threshold is greater than 0, a specific value of the quantity-of-time threshold may be set by a skilled person based on an actual requirement. This is not excessively limited herein. For example, the specific value may be set to 1 or 2.

The physiological data collection method provided in embodiments of this application may be applied to wearable devices such as a band, a watch, and a ring. In this case, the wearable device is an execution body of the physiological data collection method provided in embodiments of this application.

In the following embodiments of this application, an example in which the first sensor is an optical heart rate sensor is used for solution description. Therefore, a principle of the PPG is first described herein as follows:

PPG is a non-invasive detection and analysis method for detecting a blood volume change of a user by using photoelectric means. The optical heart rate sensor includes a light emitter (which may also be referred to as a PPG lamp, and may be a light emitting device such as a light emitting diode) and a photosensitive sensor (which is also referred to as a photoelectric receiver). During detection, the PPG lamp keeps enabled, to irradiate a light beam of a wavelength to skin of a user. After reaching a skin surface, the light beam is received by the photosensitive sensor in a transmission or reflection manner. In this process, light intensity received by the photosensitive sensor is weakened by absorption and attenuation of skin muscles and blood. Light absorption of the skin muscles, bones, and the like remains basically unchanged in a whole blood circulation process. However, a blood volume in skin changes pulsatingly under an action of a heart. Therefore, the light intensity received by the photosensitive sensor also changes pulsatingly. After receiving the light beam, the photosensitive sensor converts a light intensity change into an electrical signal, so that data of a blood volume change can be obtained. Then, the electrical signal is converted into a digital signal, so that a PPG signal used for sleep monitoring can be obtained. Finally, the PPG signal is further extracted and analyzed by using an analysis method such as a frequency domain analysis method or a time domain analysis method, so that a biometric data analysis result such as blood oxygen and pulse rate in a long-time sleep process of the user can be obtained.

It can be learned from the foregoing description that, when the first sensor is an optical heart rate sensor, enabling the optical heart rate sensor essentially means enabling the PPG lamp. Disabling the optical heart rate sensor means disabling the PPG lamp. Power consumption is high when the PPG lamp keeps enabled. Therefore, in embodiments of this application, an occasion for enabling the PPG lamp needs to be controlled, to reduce power consumption for collecting physiological data.

In addition, in embodiments of this application, application cases are classified into two types based on whether historical sleep data of long-time sleep of the user can be obtained.

Case 1: The wearable device cannot obtain historical sleep data of long-time sleep of the user.

Case 2: The wearable device can obtain historical sleep data of long-time sleep of the user.

Case 1 mainly occurs in a period of time after the user purchases the wearable device, or a period of time after data of the wearable device is cleared. In this case, the user uses the wearable device for a small quantity of times, and may even have not been subject to sleep monitoring. As a result, the wearable device cannot obtain historical sleep data of long-time sleep of the user. Case 2 mainly occurs after the user uses the wearable device for a period of time. In this case, the wearable device has recorded historical sleep data of the user within a period of time.

For Case 1, in embodiments of this application, whether to enable the first sensor may be determined based on whether the user falls asleep and a long-time sleep status of the user.

For Case 2, in embodiments of this application, whether to enable the first sensor may be determined based on whether the user falls asleep and a long-time sleep status of the user. Alternatively, whether to enable the first sensor may be determined based on whether the user falls asleep, a falling-asleep moment, and a long-time sleep status.

Herein, a physiological data collection technical solution corresponding to Case 1 is first described by using a specific embodiment. In a specific embodiment, it is assumed that the first sensor is an optical heart rate sensor.

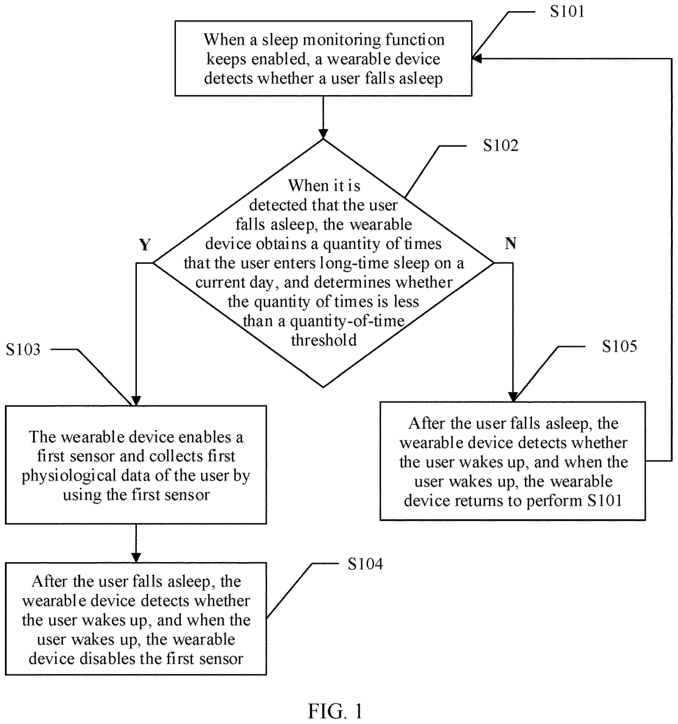

FIG. 1 shows an implementation flowchart of a physiological data collection method according to an embodiment of this application. Details of S101 to S105 are described as follows.

S101: When a sleep monitoring function keeps enabled, a wearable device detects whether a user falls asleep.

In this embodiment of this application, when the sleep monitoring function keeps enabled, the wearable device detects whether the user enters sleep (that is, falls asleep). A sensor and a detection method used for falling-asleep detection are not excessively limited in this embodiment of this application, and may be set by a skilled person based on an actual requirement. In some optional embodiments, to reduce power consumption for falling-asleep detection, some motion sensors that can detect motion data of a user may be selected to detect whether the user falls asleep. In other words, whether the user falls asleep is identified based on the motion data detected by the motion sensors. For example, an accelerometer (accelerometer, ACC) may be used to collect a user action signal, and identify, based on the collected action signal, whether the user is in a falling-asleep state. For example, it may be determined that the user falls asleep when an action of the user is small within a period of time.

S102: When it is detected that the user falls asleep, the wearable device obtains a quantity of times that the user enters long-time sleep on a current day, and determines whether the quantity of times is less than a quantity-of-time threshold. When the quantity of times is less than the quantity-of-time threshold, S103 is performed. When the quantity of times is greater than or equal to the quantity-of-time threshold, S105 is performed.

In this embodiment of this application, a quantity of times (that is, a first quantity of times) that the user enters long-time sleep each day (that is, each user activity day) is recorded. To be specific, each time it is detected that the user performs long-time sleep, the quantity of times is increased by one. When a new user activity day starts, the quantity of times is reset to zero (that is, an initial value of the first quantity of times is 0). It should be noted that a manner of recording the quantity of times is not limited in this embodiment of this application, and may be set by a skilled person. For example, a parameter may be set to record a real value of the quantity of times, for example, 0, 1, or 2. Alternatively, a flag bit having at least two states may be set, and different states of the flag bit are used to represent different quantity-of-time values. In this case, each change of the quantity of times corresponds to a state change of the flag bit. For example, a bit may be set as the flag bit. In this case, two states may be recorded: 0 and 1. The two states may be respectively associated with two quantity-of-time values. For example, the state 0 corresponds to a quantity-of-time value 0, and the state 1 corresponds to a quantity-of-time value 1. In addition, a method for detecting that the user enters long-time sleep each day is not limited herein, and may be set by a skilled person based on an actual requirement. For example, sleep duration is detected each time the user falls asleep, and sleep whose duration is greater than or equal to a duration threshold is determined as long-time sleep.

When it is detected that the user falls asleep, a quantity of times that the user enters long-time sleep on a current day (that is, a current user activity day) is obtained in this embodiment of this application. In other words, the quantity of times that the user enters long-time sleep on the current user activity day is determined by reading a recorded quantity of times, identifying a state of the flag bit, or the like. In an optional embodiment of this application, to distinguish the current user activity day from a previous activity day, a time period that has passed in the current user activity day may be referred to as a first time period. In this case, S102 is as follows:

When it is detected that the user falls asleep, the wearable device obtains the first quantity of times that the user enters long-time sleep within the first time period.

For example, it is assumed that the user activity day is set to 8:00 p.m. on each calendar day to a moment before 8:00 p.m. on a next calendar day. In addition, it is assumed that the user is detected to fall asleep at 10:00 p.m. on August 13, 2020. In this case, the first time period refers to a period lasting from 8:00 p.m. to 10:00 p.m. on August 13, 2020. In other words, a quantity of times that the user enters long-time sleep within this period of time needs to be obtained. However, when it is detected that the user sleeps before 8 p.m., the first time period needs to be determined forward. For example, it is assumed that the user is detected to fall asleep at 5:00 p.m. on August 13, 2020. In this case, the first time period refers to a period lasting from 8:00 p.m. on August 12, 2020 to 5:00 p.m. on August 13, 2020. In this case, the first time period crosses a calendar day.

In an optional embodiment of this application, the first time period may alternatively be a time period before a current moment. In this case, the first time period may be associated with or not associated with a user activity day. Specifically, a skilled person may set a range of the first time period based on an actual requirement. For example, the range may be set to a past period of time, for example, 24 hours. In this case, a quantity of times that the user performs long-time sleep within the past period of time may also be evaluated, and whether to enable the first sensor is determined based on the quantity of times.

After the quantity of times that the user enters long-time sleep on a current day is determined, in this embodiment of this application, it is determined whether the quantity of times reaches the preset quantity-of-time threshold. When the quantity of times does not reach the preset quantity-of-time threshold, it indicates that a quantity of times that the user enters long-time sleep on the current day is small, and it is of a great possibility that the user enters long-time sleep this time. Therefore, in this case, an operation of enabling a first sensor in S103 is performed, to collect physiological data during the long-time sleep.

On the contrary, when the quantity of times that the user enters long-time sleep on the current day reaches the quantity-of-time threshold, that is, the quantity of times is greater than or equal to the quantity-of-time threshold, it indicates that the user has entered long-time sleep for a large quantity of times on the current day. Therefore, it is of a great possibility that the sleep is short-time sleep. Therefore, in this case, an operation of enabling a first sensor in S103 is not performed.

In an optional embodiment of this application, it is considered that in an actual case, on a single user activity day, when the user falls asleep for the first time, a quantity of times that the user previously enters long-time sleep is 0, and is definitely less than the quantity-of-time threshold. Therefore, for first falling asleep every day, theoretically, it may not be necessary to obtain the first quantity of times, but the first quantity of times is directly determined to be less than the quantity-of-time threshold. In this case, S102 may be replaced with the following.

When it is detected that the user falls asleep, identify whether the falling asleep is the first time of falling asleep within the first time period; and
when the falling asleep is the first time of falling asleep, determine that the first quantity of times is less than the quantity-of-time threshold.

In this embodiment of this application, when it is detected that the user falls asleep, it is identified whether the falling asleep is the first time that the user falls asleep within the first time period. If the falling asleep is the first time that the user falls asleep, a specific value of a quantity of times that the user enters long-time sleep may not be obtained (that is, regardless of the value of the first quantity of times); instead, it is determined that the quantity of times is less than the quantity-of-time threshold, and physiological data collection in a next step is started.

Correspondingly, when the falling asleep is not the first time of falling asleep within the first time period, an original logic of S 102 is performed as follows: The wearable device obtains a quantity of times that the user enters long-time sleep on the current day, determines whether the quantity of times is less than the quantity-of-time threshold, and performs processing.

In an optional embodiment of this application, after it is detected that a quantity of times that the user enters long-time sleep reaches the quantity-of-time threshold, on the current user activity day, the quantity of times that the user enters long-time sleep is not updated in this embodiment of this application regardless of whether the user subsequently enters long-time sleep. In other words, when the first quantity of times is equal to the quantity-of-time threshold, the first quantity of times on the current user activity day is not updated. Not updating means that when the first quantity of times is recorded by using a specific value, the value of the first quantity of times no longer increases. When the first quantity of times is recorded by using a flag bit, a state of the flag bit no longer changes.

When the quantity of times that the user enters long-time sleep reaches the quantity-of-time threshold, it is considered in this embodiment of this application that the user no longer performs long-time sleep on the current user activity day. Therefore, the first quantity of times is not updated subsequently, so that a workload of performing long-time sleep identification and a workload of recording a quantity of times can be reduced.

S103: The wearable device enables the first sensor and collect first physiological data of the user by using the first sensor.

When it is determined that a quantity of times that the user enters long-time sleep on the current user activity day does not reach the quantity-of-time threshold, it indicates that the sleep of the user is of a great possibility to be long-time sleep. Therefore, in this case, the wearable device enables a PPG lamp, and collects physiological data of the user by using the PPG lamp and a photosensitive sensor. In this embodiment of this application, the physiological data collected this time is referred to as the first physiological data.

S104: After the user falls asleep, the wearable device detects whether the user wakes up, and when the user wakes up, the wearable device disables the first sensor.

S105: After the user falls asleep, the wearable device detects whether the user wakes up, and when the user wakes up, the wearable device returns to perform S 101, to continue to monitor whether the user falls asleep.

After the user falls asleep, the wearable device continues to monitor whether the user wakes up from sleep, that is, whether the sleep ends. For a case in which the PPG lamp is not enabled, there is no requirement for disabling the PPG lamp, and corresponding physiological data is not collected. Therefore, in this embodiment of this application, the wearable device returns to perform the operation in S101 and continues to perform sleep monitoring on the user, to start detection for next sleep of the user.

For a case in which the PPG lamp needs to be enabled, the wearable device disables the PPG lamp, to end collection for physiological data of the user, and obtain physiological data in a current sleep process. A sensor and a detection method used for monitoring waking up of the user are not excessively limited in this embodiment of this application, and may be set by a skilled person based on an actual requirement. In some optional embodiments, to reduce power consumption for waking-up detection, some motion sensors that can detect motion data of a user may be selected to detect whether the user wakes up. In other words, whether the user wakes up is identified based on the motion data detected by the motion sensors. For example, an ACC may be used to collect a user action signal, and identify, based on the collected action signal, whether the user is in a monitored state. For example, after the user falls asleep, when a case in which the user moves for a large amplitude and the motion lasts for a period of time is detected, it is determined that the user wakes up.

After the PPG lamp is disabled, in one aspect, in this embodiment of this application, the wearable device may return to perform the operation in S101 and continue to perform sleep monitoring on the user, to start identification for next sleep of the user.

In another aspect, physiological data during the sleep has been collected, and a falling-asleep moment and a waking-up moment of the sleep are known. Therefore, sleep duration (that is, first duration) may be first determined based on the falling-asleep moment and the waking-up moment. When the sleep duration is greater than or equal to the duration threshold, it indicates that the sleep is long-time sleep. When the sleep duration is less than the duration threshold, it indicates that the sleep is short-time sleep. In this case, the physiological data collected this time may be discarded, to save storage space of the wearable device.

For a case in which the sleep is long-time sleep, the following operations may be performed.

Operation 1: Update the quantity of times that the user enters long-time sleep on the current user activity day.

In this embodiment of this application, the quantity of times that the user actually enters long-time sleep on the current user activity day needs to be recorded, to determine an actual long-time sleep state of the user and determine whether the first sensor needs to be enabled when it is detected that the user falls asleep. Therefore, when it is identified that the sleep is long-time sleep, the quantity of times is updated in this embodiment of this application, to update the quantity of times in real time. When it is selected to record a real value of the quantity of times by using a parameter, updating the quantity of times refers to adding 1 to the real value. When the quantity of times is recorded by using a flag bit, updating the quantity of times refers to updating a state of the flag bit to a state corresponding to the original quantity of times added by 1. For example, it is assumed that a bit is set to the flag bit, a state 0 corresponds to a quantity-of-time value 0, and a state 1 corresponds to a quantity-of-time value 1. In addition, it is assumed that before the sleep, a quantity of times that the user performs long-time sleep on the current user activity day is 0. In this case, a state of the bit needs to be changed to a state corresponding to 0+1=1, that is, changed to the state 1.

In an optional embodiment of this application, when it is set that after it is detected that a quantity of times that the user enters long-time sleep reaches the quantity-of-time threshold, on the current user activity day, the quantity of times that the user enters long-time sleep is not updated regardless of whether the user subsequently enters long-time sleep. In this case, updating the quantity of times refers to keeping a current situation of the quantity of times.

It should be noted that, in a case in which the first time period is not the current user activity day, Operation 1 may be replaced with the following: Update the quantity of times that the user enters long-time sleep within the first time period. A principle and operation details are basically the same as those in Operation 1. Details are not described herein again.

Operation 2: Analyze the physiological data collected this time, to obtain corresponding sleep analysis data, so that monitoring for the long-time sleep is implemented.

To implement monitoring for long-time sleep of the user, in this embodiment of this application, after it is determined that the sleep is long-time sleep, the physiological data collected during the long-time sleep may be analyzed, to obtain a corresponding analysis result. A manner for analyzing the physiological data and specific content of the analysis result are not excessively limited herein, and may be set by a skilled person based on an actual situation. In addition, the analyzed physiological data may be locally stored in the wearable device or locally deleted.

In this embodiment of this application, for a case in which the wearable device cannot obtain historical sleep data of long-time sleep of the user, the wearable device does not continuously enable a sensor during a period in which sleep monitoring keeps enabled, but continuously detects whether the user falls asleep. When it is detected that the user falls asleep, it is determined whether a quantity of times that the user is in long-time sleep on the current activity day (or the first time period) reaches the quantity-of-time threshold. In addition, the sensor is enabled only when the quantity-of-time threshold is not reached, to collect physiological data of the user. In addition, when the user is in sleep, waking-up monitoring continues to be performed on the user, and the sensor is disabled when it is detected that the user wakes up.

It is considered that a quantity of times that the user performs long-time sleep within a specific time period in actual life is very limited. Therefore, in this embodiment of this application, whether to enable the sensor is determined based on two conditions: whether the user falls asleep and whether a quantity of long-time sleep times within a recent period of time reaches the quantity-of-time threshold. The sensor is enabled when the quantity of times does not reach the quantity-of-time threshold. Compared with that in a related technology, a case in which the sensor keeps enabled in a time period in which the user is awake is avoided. In normal life and work, the user is in a sober state most of the time. Therefore, compared with keeping a related sensor enabled all day, this embodiment of this application can reduce a large amount of sensor collection work and power consumption. Compared with fixedly keeping a sensor enabled in a preset time period, this can better adapt to an actual sleep habit of the user, and reduces cases in which physiological data of the user in an awake state is collected. In addition, setting the quantity-of-time threshold can effectively avoid a case in which physiological data collection is performed on all sleep periods of the user, and makes it less probable to enable a related sensor for short-time sleep. Therefore, power consumption for physiological data collection can be reduced. In conclusion, in embodiments of this application, physiological data collection performed when the user is awake can be avoided, and cases in which physiological data collection is performed when the user is in short-time sleep can be reduced. Physiological data can be accurately collected during long-time sleep. Therefore, a workload of collecting physiological data during long-time sleep by the sensor can be reduced as well as power consumption, for lower electricity consumption and a longer battery life of the wearable device.

In addition, when the PPG lamp needs to be enabled, precisely enabling the PPG lamp can avoid interference caused by the PPG lamp to the user when the user performs short-time sleep, so that sleep quality of the user is improved.

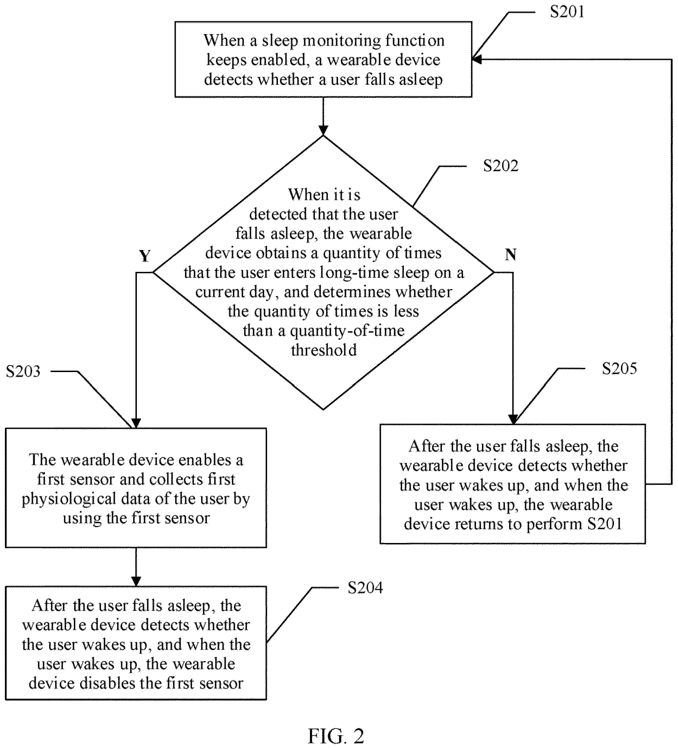

For the foregoing Case 2, the wearable device may obtain a physiological data collection solution corresponding to the historical sleep data of long-time sleep of the user. This is described by using a specific embodiment. In a specific embodiment, it is assumed that the first sensor is an optical heart rate sensor. FIG. 2 shows an implementation flowchart of a physiological data collection method according to an embodiment of this application. Details are described as follows.

S201: When a sleep monitoring function keeps enabled, a wearable device detects whether a user falls asleep.

S202: When it is detected that the user falls asleep, the wearable device obtains a quantity of times that the user enters long-time sleep on a current day, and determines whether the quantity of times is less than a quantity-of-time threshold. When the quantity of times is less than the quantity-of-time threshold, S203 is performed. When the quantity of times is greater than or equal to the quantity-of-time threshold, S205 is performed.

S203: The wearable device enables the first sensor and collect first physiological data of the user by using the first sensor.

S204: After the user falls asleep, the wearable device detects whether the user wakes up, and when the user wakes up, the wearable device disables the first sensor.

S205: After the user falls asleep, the wearable device detects whether the user wakes up. When the user wakes up, the wearable device returns to perform S201, to continue to monitor whether the user falls asleep.

Principles, operation details, beneficial effects, and the like of this embodiment of this application are the same as those of the embodiment shown in FIG. 1. Therefore, for details, refer to related descriptions of the embodiment shown in FIG. 1. Details are not described herein again. In addition, some detailed, optimized, or extended embodiments related to the embodiment shown in FIG. 1 may also be combined with this embodiment of this application for application. For example, embodiments in which the quantity of times of long-time sleep of the user is updated, the collected physiological data is analyzed, and the like may also be applied with reference to this embodiment of this application. For specific details of these embodiments, refer to the related descriptions of the embodiment shown in FIG. 1. Details are not described herein again.

In this embodiment of this application, although the wearable device can obtain the historical sleep data of long-time sleep of the user, the wearable device can choose not to use the historical sleep data. The choice is to perform processing in a same processing manner as that in Case 1: The wearable device cannot obtain historical sleep data of long-time sleep of the user.

For Case 2, FIG. 3 shows an implementation flowchart of another physiological data collection method according to an embodiment of this application. It is considered that in actual life, long-time sleep is a regular activity for the user. Generally, a user falls asleep each day at a regular moment. Based on this, in this embodiment of this application, a falling-asleep moment range (that is, a second time period) in which the user generally enters long-time sleep is determined based on an actual long-time sleep status of the user. When sleep monitoring is performed, three conditions are simultaneously used as determining conditions for enabling a sensor: whether the user falls asleep, whether a falling-asleep moment belongs to the falling-asleep time period, and whether a quantity of long-time sleep times on a current user activity day reaches a quantity-of-time threshold. Details are described as follows.

S301: When a sleep monitoring function keeps enabled, a wearable device detects whether a user falls asleep.

Operation details in S301 are the same as those in S101. For details, refer to the descriptions in S101. Details are not described herein again.

S302: When it is detected that the user falls asleep, the wearable device identifies whether a falling-asleep moment is within the second time period.

It is considered that a user actually performs long-time sleep regularly. For example, most users fall asleep at night. Even for some users who need a daytime rest due to a work or life requirement, it is regular for the users to perform long-time sleep to rest in the daytime. Therefore, in this embodiment of this application, a falling-asleep moment range in which the user enters long-time sleep on a daily basis is determined in advance based on an actual long-time sleep status of the user. "In advance" refers to a moment before an operation of "identifying whether a falling-asleep moment is within the second time period" is performed.

A specific case of the falling-asleep moment range is not limited in this embodiment of this application, and may be determined by a skilled person or a user based on an actual user situation. For example, in some optional embodiments, a skilled person or a user may manually set a falling-asleep moment range based on an actual falling-asleep status of long-time sleep of the user. For example, the range may be set to a period lasting from 8:00 p.m. to 12:00 p.m. each day. Alternatively, in some other optional embodiments, the wearable device may perform analysis based on historical sleep data of the user, to obtain the falling-asleep moment range of the user (in this case, the second time period is time period data determined based on the historical sleep data of the user). It should be noted that the falling-asleep moment range may be a single time period, or may be a plurality of different time periods. This is not specifically limited herein. For example, the range may refer to a period lasting from 11 a.m. to 1 p.m. and a period lasting from 10 p.m. to 12 p.m. each day.

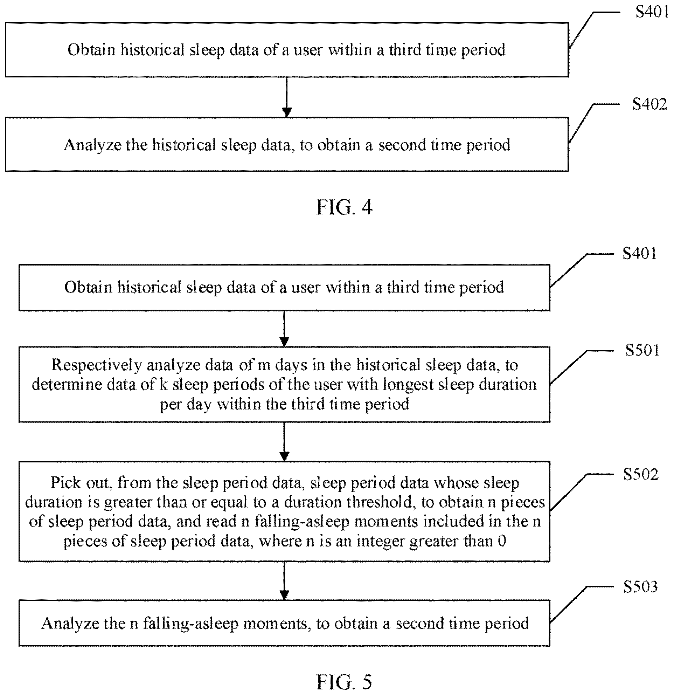

In an optional embodiment of this application, as shown in FIG. 4, an operation of determining the falling-asleep moment range includes the following steps.

S401: Obtain historical sleep data of the user within a third time period.

S402: Analyze the historical sleep data, to obtain the second time period.

In this embodiment of this application, historical sleep data of the user within a past time period (that is, the third time period) is obtained. The historical sleep data includes falling-asleep moment data of the user per day within a past period of time, that is, a falling-asleep moment of the user per day. The historical sleep data may be stored locally in the wearable device, an external memory, or another device that can exchange data with the wearable device. Precision of the falling-asleep moment data is not excessively limited in this embodiment of this application, and may be set by a skilled person based on an actual requirement. For example, when the precision is measured by hour, the falling-asleep moment data records a specific time point at which the user falls asleep each day. When the precision is measured by minute, a specific time point and a specific minute at which the user falls asleep each day are recorded. In this embodiment of this application, the "each day" corresponding to the historical sleep data may be each calendar day, or may be each user activity day.

Based on the obtained historical sleep data, in this embodiment of this application, the historical sleep data and the falling-asleep moment data thereof is analyzed, to obtain a falling-asleep moment range in which the user enters long-time sleep on a daily basis. A specific method for analyzing the falling-asleep moment data is not excessively limited in this embodiment of this application, and may be set by a skilled person based on an actual requirement. For example, in some optional embodiments, data corresponding to long-time sleep of the user may be picked out based on the historical sleep data, then falling-asleep moments of long-time sleep within each day is determined from the data, and an upper limit and a lower limit of the falling-asleep moments are used to obtain the falling-asleep moment range.

In an optional embodiment of this application, it is assumed that the third time period includes m days, the quantity-of-time threshold is k, and both m and k are integers greater than 0. As shown in FIG. 5, an operation in S402 specifically includes S501 to S503.

S501: Respectively analyze data of m days in the historical sleep data, to determine data of k sleep periods of the user with longest sleep duration per day within the third time period.

In this embodiment of this application, a quantity -of-time threshold k is set for long-time sleep performed by the user each day. Therefore, when the historical sleep data is processed, sleep period data corresponding to each time of sleep of the user within each day is first identified. In addition, sleep period data of k times of sleep is sequentially picked out from sleep period data of each day in a descending order of sleep duration (that is, duration lasting from a falling-asleep moment to a waking-up moment), to obtain k pieces of sleep period data. In this case, when a total quantity of pieces of sleep period data in a single day is less than k, all sleep period data within the day is extracted. When k=1, step 1 is performed to determine, from the historical sleep data, sleep period data of sleep with longest sleep duration of the user per day within the third time period. After step 1 is performed, a maximum of m×k pieces of sleep period data can be obtained.

S502: Pick out, from the sleep period data, sleep period data whose sleep duration is greater than or equal to a duration threshold, to obtain n pieces of sleep period data, and read n falling-asleep moments included in the n pieces of sleep period data, where n is an integer greater than 0.

After the sleep period data is obtained through picking, in this embodiment of this application, whether sleep duration corresponding to each piece of sleep period data reaches the duration threshold continues to be determined, and sleep period data that reaches the duration threshold is picked out. Further, sleep period data of long-time sleep actually performed by the user each day is obtained. Then, specific falling-asleep moments are read from the sleep period data. In this way, a moment at which the user actually enters long-time sleep each day can be obtained.

It should be noted that, for a single day, a quantity of pieces of sleep period data that meets the duration threshold may be any value from 0 to k. When the quantity is 0, it indicates that the user does not perform long-time sleep within the day. In this case, the user may stay up late or the like. For ease of description, in this embodiment of this application, a total quantity value of the sleep period data that meets the duration threshold is set to n.

In this embodiment of this application, step 1 and step 2 are used to extract falling-asleep moments (that is, falling-asleep moment data, and in this embodiment of this application, the falling-asleep moment data includes n falling-asleep moments) at which the user enters long-time sleep each day within the third time period.

S503: Analyze the n falling-asleep moments, to obtain the second time period.

After the n falling-asleep moments that can be used for reference are obtained, these falling-asleep moments are analyzed in this embodiment of this application, to determine a rule of a falling-asleep behavior of the user. An analysis method may be as follows: Extremum values of the n falling-asleep moments that can be used for reference are obtained, a modal number of the n falling-asleep moments is obtained, cluster analysis is performed, or the like.

Examples of several optional analysis methods are described as follows.

Method 1: An earliest moment and a latest moment (two extremum values) among the n falling-asleep moments are respectively used as a start moment and an end moment of a falling-asleep moment range, to determine the falling-asleep moment range. Each falling-asleep moment is a time point in a single day. Therefore, when comparison is performed in a unit of a single day, the falling-asleep moments are in a sequence. In this embodiment of this application, the earliest falling-asleep moment is used as a start moment, and a latest falling-asleep moment is used as an end moment, to determine the corresponding falling-asleep moment range. For example, it is assumed that there are four falling-asleep moments in total: 8:15 p.m., 9:00 p.m., 9:10 p.m., and 10:00 p.m.. In this case, the earliest moment is 8:15 p.m. and the latest moment is 10:00 p.m.. Therefore, the falling-asleep moment range is from 8:15 p.m. to 10:00 p.m..

Method 2: In this case, an average value u of the n falling-asleep moments is first calculated.

Then, a standard deviation p of the n falling-asleep moments is calculated.

Finally, T1=u-b×p and T2=u+b×p are calculated, and a start moment of a falling-asleep moment range is set to T1, and an end moment is set to T2. In this method, b is a constant term coefficient, and b>0. A specific value may be set by a skilled person based on an actual requirement. Theoretically, a larger value of b indicates a larger falling-asleep moment range.

Method 3: Cluster analysis is performed on the n falling-asleep moments by using some clustering algorithms, to obtain a falling-asleep moment range. For example, the clustering algorithm may be a K-means algorithm, an AP clustering algorithm, or a neural network-based clustering model.

In an optional embodiment of this application, it is considered that duration of long-time sleep of each person may be different in actual application. Therefore, to make the duration threshold more suitable for an actual sleep habit of the user, after S502 is performed, in this embodiment of this application, the existing duration threshold is updated based on the obtained historical sleep data. Details are described as follows.

S504: Obtain n pieces of sleep duration corresponding to the n pieces of sleep period data, and update the duration threshold based on the n pieces of sleep duration.

A specific method for updating the duration threshold is not limited in this embodiment of this application, and may be set by a skilled person. For example, shortest sleep duration among the n pieces of sleep duration may be used as the duration threshold, or a modal number or an average value of the n pieces of sleep duration may be used as the duration threshold.

In this embodiment of this application, the duration threshold is updated by using the historical sleep data, so that a value of the duration threshold can be adaptive to an actual sleep habit of the user. Therefore, in a process of identifying long-time sleep of the user, accuracy of identifying long-time sleep of the user can be higher, so that time for enabling the sensor is selected more accurately, and possibilities of enabling the sensor by mistakes are reduced. Therefore, power consumption for collecting physiological data of the user during long-time sleep can be reduced.

S303: When the falling-asleep moment of the user is within the second time period, the wearable device obtains a quantity of times that the user enters long-time sleep on a current day, and determines whether the quantity of times is less than a quantity-of-time threshold. When the quantity of times is less than the quantity-of-time threshold, S304 is performed. When the quantity of times is greater than or equal to the quantity-of-time threshold, S306 is performed.

When it is determined that the falling-asleep moment of the user is within the falling-asleep moment range, it indicates that the sleep is of a great possibility to be long-time sleep. Therefore, in this case, in this embodiment of this application, the quantity of times that the user performs long-time sleep on the current day is determined. Operation details, principles, beneficial effects, and the like of determining the quantity of times of long-time sleep are basically the same as those in S102. For details, refer to the related descriptions in S102. Details are not described herein again.

It should be noted that the "current day" in S303 may alternatively be replaced with the "first time period". For description of the first time period, refer to the description in S102. Details are not described herein again.

S304: The wearable device enables the first sensor and collect first physiological data of the user by using the first sensor.

S305: After the user falls asleep, the wearable device detects whether the user wakes up, and when the user wakes up, the wearable device disables the first sensor.

S306: After the user falls asleep, the wearable device detects whether the user wakes up. When the user wakes up, the wearable device returns to perform S301, to continue to monitor whether the user falls asleep.

Operations in S304 to S306 are the same as those in S103 to S105. For details, refer to the descriptions in S103 to S105. Details are not described herein again.

In addition, some detailed, optimized, or extended embodiments related to the embodiment shown in FIG. 1 may also be combined with this embodiment of this application for application. For example, embodiments in which the quantity of times of long-time sleep of the user is updated, the collected physiological data is analyzed, and the like may also be applied with reference to this embodiment of this application. For specific details of these embodiments, refer to the related descriptions of the embodiment shown in FIG. 1. Details are not described herein again.

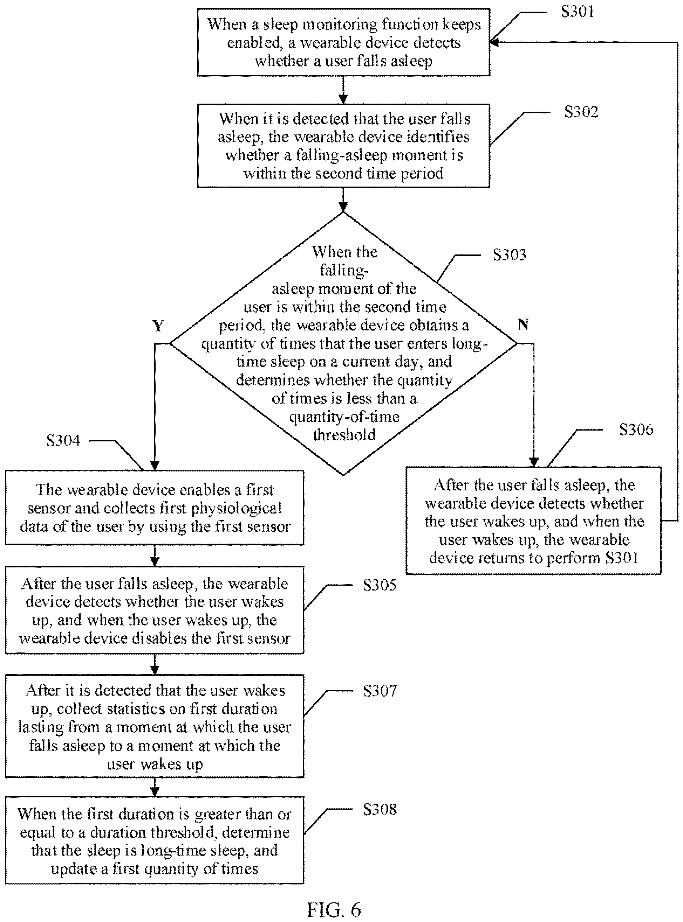

For example, corresponding to Operation 1 in the embodiment shown in FIG. 1, as shown in FIG. 6, after S305 is performed, this embodiment of this application may further include the following steps.

S307: After it is detected that the user wakes up, collect statistics on first duration lasting from a moment at which the user falls asleep to a moment at which the user wakes up.

S308: When the first duration is greater than or equal to a duration threshold, determine that the sleep is long-time sleep, and update the first quantity of times.

Physiological data during the sleep has been collected, and a falling-asleep moment and a waking-up moment of the sleep are known. Therefore, sleep duration (that is, first duration) may be first determined based on the falling-asleep moment and the waking-up moment. When the sleep duration is greater than or equal to the duration threshold, it indicates that the sleep is long-time sleep. When the sleep duration is less than the duration threshold, it indicates that the sleep is short-time sleep. In this case, the physiological data collected this time may be discarded, to save storage space of the wearable device. In a case in which the sleep is long-time sleep, the quantity of times of long-time sleep of the user may be updated.

It should be noted that, in a case in which the first time period is not the current user activity day, S308 may be replaced with the following: When the first duration is greater than or equal to the duration threshold, determine that the sleep is long-time sleep, and update the quantity of times that the user enters long-time sleep within the first time period. A principle and operation details are basically the same as those in S308. Details are not described herein again.

The duration threshold used in S308 is a latest duration threshold. In other words, when the duration threshold is updated in the manner described in S504, the latest updated duration threshold is used in this case.

In addition, it should be noted that, after S305 is performed, in this embodiment of this application, the physiological data of the user during the sleep may be obtained. When the sleep duration is greater than or equal to the duration threshold (that is, it is identified after S307 is performed that the first duration is greater than or equal to the duration threshold), it indicates that the sleep is long-time sleep. Correspondingly, a falling-asleep moment and a waking-up moment of the sleep also become a part of historical sleep data. To make the falling-asleep moment range accurate in real time, so as to adapt to a sleep habit of the user that may change in different periods (for example, in different seasons, sleep habits are different and falling-asleep moments are different, and even within a month, a sleep habit at the beginning of the month and a sleep habit at the end of the month may be different), after S305 is performed, this embodiment of this application may further include the following steps.

S309: After it is detected that the user wakes up, collect statistics on first duration lasting from a moment at which the user falls asleep to a moment at which the user wakes up.

S310: When the first duration is greater than or equal to the duration threshold, perform the operation of obtaining the historical sleep data of the user within the third time period in S401.

In this case, operations in S401 and S402 may be triggered (in combination with the embodiment shown in FIG. 5, operations in S501 to S503 are further triggered), to update the falling-asleep moment range in a timely manner. In addition, when this embodiment of this application is applied in combination with FIG. 6, S309 is S307.

In an optional embodiment of this application, it is considered that the sleep habit includes a falling-asleep moment, and further includes sleep duration. In other words, long-time sleep duration of the user may vary in different periods. Therefore, after S309 is performed, in combination with the embodiment shown in FIG. 5, this embodiment of this application may further include the following steps.

When the first duration is greater than or equal to the duration threshold, the operation of obtaining n pieces of sleep duration corresponding to the n pieces of sleep period data and updating the duration threshold based on the n pieces of sleep duration in S504 is performed.

In this embodiment of this application, each time after a latest falling-asleep moment and sleep duration of the long-time sleep are obtained, timely update of the falling-asleep moment range and the duration threshold can be triggered in a timely manner in this embodiment of this application. In this way, this embodiment of this application can automatically adapt to a change of a long-time sleep habit of the user in different periods. Therefore, more accurate identification for long-time sleep can be implemented, and possibilities of enabling a sensor by mistakes can be reduced. Power consumption for collecting physiological data of the user during long-time sleep can be reduced.

In an optional embodiment of this application, operations in S301 to S306 can be performed to theoretically accurately identify long-term sleep of the user. However, in actual application, it is found that the following scenario may exist: Although the user wants to fall asleep within a falling-asleep moment range and perform long-time sleep, the sleep of the user is interrupted due to interference from an external factor (for example, being woken up by noise such as a call or an alarm clock). In this case, the user generally wants to re-enter long-time sleep. The sleep duration may be less than the duration threshold, and a next falling-asleep moment of the user may be beyond the falling-asleep moment range. In this case, a case in which the user actually wants to perform long-time sleep but a sensor cannot be identified and enabled occurs.

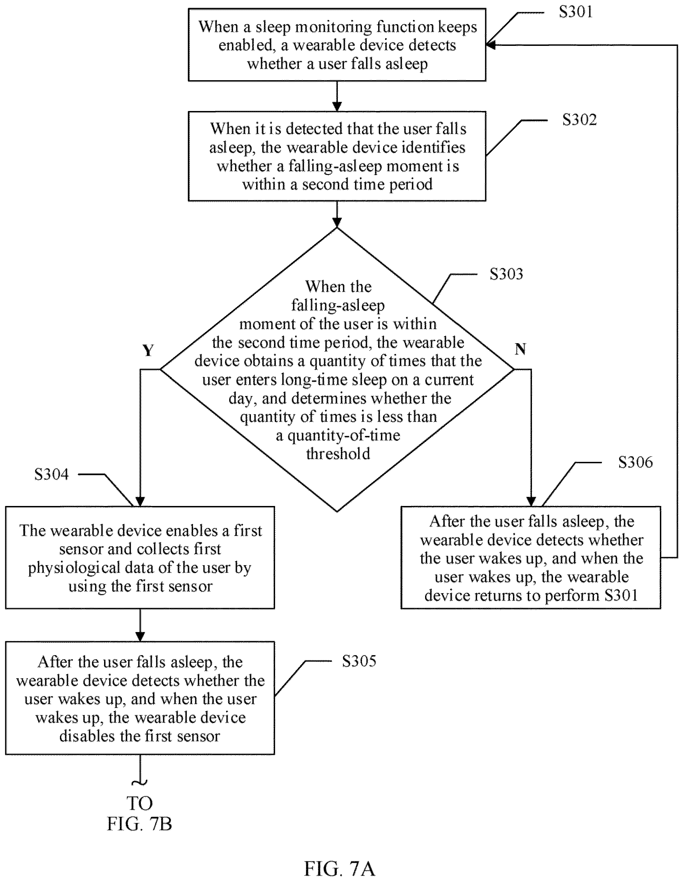

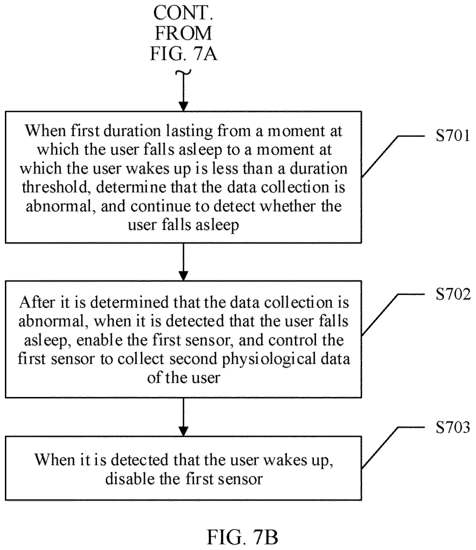

An example is used for description. It is assumed that a falling-asleep moment range is from 8:00 p.m. to 11:00 p.m., and a duration threshold is 6 hours. In addition, it is assumed that a user A falls asleep at 10:30 p.m., and a quantity of long-time sleep times on a current day is less than a quantity-of-time threshold. It is assumed that after the user falls asleep at 10:30 p.m., the user is awakened by noise, and sleep duration of the user is less than 6 hours. For example, the user is awakened by a call at 11:30 p.m.. In this case, the user generally performs long-time sleep again after waking up. It is assumed that the re-falling-asleep moment is beyond the falling-asleep moment range. For example, it is assumed that the user finishes answering the call at 11:50 p.m. and then performs long-time sleep again. In this case, it cannot be identified, by using the operations in S301 to S306, that the user intends to perform long-time sleep this time. To resolve this problem, as shown in FIG. 7A and FIG. 7B, after S305 is performed, this embodiment of this application further includes S701 to S703.

S701: When the first duration lasting from a moment at which the user falls asleep to a moment at which the user wakes up is less than the duration threshold, determine that the data collection is abnormal, and continue to monitor whether the user falls asleep.

When the sleep duration of the user is less than the duration threshold, it indicates that the user is of a great possibility to wake up because of impact of an external factor. In this case, in this embodiment of this application, it is determined that the collection for physiological data of the user is abnormal.

S702: After it is determined that the data collection is abnormal, when it is detected that the user falls asleep, enable a first sensor, and control the first sensor to collect second physiological data of the user.

When the collection for physiological data of the user is abnormal, in this embodiment of this application, when the user falls asleep next time, whether to enable the sensor is not determined based on operations in S302 and S303, but the sensor is directly enabled. To be specific, when the user falls asleep next time, regardless of whether a falling-asleep moment is within the falling-asleep moment range, the sensor is enabled in this embodiment of this application, and collection for physiological data of the user is performed. According to this embodiment of this application, even if the user is interrupted by an interference factor such as noise during long-time sleep, physiological data collection can be performed on the user timely when the user re-enters long-time sleep. In this way, physiological data collection performed during long-time sleep is more accurate and reliable. To distinguish from the physiological data collected in the embodiment shown in FIG. 3, in this embodiment of this application, physiological data collected after the user falls asleep next time is referred to as the second physiological data.

S703: When it is detected that the user wakes up, disable the first sensor.

When the user wakes up, it indicates that the sleep of the user ends. Therefore, the sensor is disabled, to end the collection for the physiological data of the user.

In this embodiment of this application, for a case in which the wearable device can obtain historical sleep data of long-time sleep of the user, in an optional manner, processing is performed by using a solution that is the same as that in FIG. 1. For specific beneficial effects in this case, refer to the descriptions of the beneficial effects in FIG. 1. Details are not described herein again.

In another optional manner, a falling-asleep moment range of an actual long-time sleep period of the user is estimated in advance based on a long-time sleep habit of the user. In a period in which sleep monitoring keeps enabled, the sensor is not continuously enabled, but falling-asleep monitoring is continuously performed on the user. When it is detected that the user falls asleep, it is determined whether a falling-asleep moment is within the falling-asleep moment range. When the falling-asleep moment is within the falling-asleep moment range, it indicates that the sleep is of a great possibility to be long-time sleep. Therefore, it is determined whether a quantity of times that the user is in long-time sleep on the current activity day (or the first time period) reaches the quantity-of-time threshold. In addition, the sensor is enabled only when the quantity -of-time threshold is not reached, to collect physiological data of the user. In addition, when the user is in sleep, waking-up monitoring continues to be performed on the user, and the sensor is disabled when it is detected that the user wakes up.

It is considered that in actual life, long-time sleep is an extremely regular behavior for the user. Therefore, in this embodiment of this application, historical sleep data of the user is analyzed to adaptively obtain a falling-asleep moment range that the user is accustomed to, so that whether the sleep of the user may be long-time sleep can be accurately distinguished. On a basis that the user falls asleep at a moment within the falling-asleep moment range of long-time sleep, that is, the user is very likely to perform long-time sleep, considering that a quantity of times that long-time sleep is performed in a specific time period is very limited, a quantity-of-time threshold is determined again. Therefore, in this embodiment of this application, whether to enable the sensor is determined based on three conditions: whether the user falls asleep, whether a falling-asleep moment is within the falling-asleep moment range that the user is accustomed to, and whether a quantity of long-time sleep times within a recent time period reaches the quantity-of-time threshold. In addition, the sensor is enabled when the falling-asleep moment is within the falling-asleep moment range that the user is accustomed to, and the quantity of times does not reach the quantity-of-time threshold. Compared with that in a related technology, a case in which the sensor keeps enabled in a time period in which the user is awake is avoided. In normal life and work, the user is in a sober state most of the time. Therefore, compared with keeping a related sensor all day, this embodiment of this application can reduce a large amount of sensor collection work and power consumption. Compared with fixedly keeping a sensor enabled in a preset time period, this can better adapt to an actual sleep habit of the user, and reduce cases in which physiological data of the user in an awake state is collected. By comparing falling-asleep moment habits of the user and setting the quantity-of-time threshold, the intent of the user each time the user falls asleep can be accurately identified, to implement accurate identification for long-time sleep. Therefore, a case in which physiological data collection is performed on all sleep periods of the user can be effectively avoided, and a probability of enabling a related sensor for short-time sleep is reduced. Power consumption for physiological data collection can be reduced.

In conclusion, in this embodiment of this application, physiological data collection performed when the user is awake can be avoided, and cases in which physiological data collection is performed when the user is in short-time sleep can be reduced. In addition, physiological data can be accurately collected during long-time sleep, and accuracy of basic data for sleep monitoring on the user is ensured. Therefore, in this embodiment of this application, a workload of collecting physiological data during long-time sleep by the sensor can be reduced, power consumption can be reduced, electricity consumption of the wearable device can be reduced, and a battery life can be prolonged. In addition, when the PPG lamp needs to be enabled, precisely enabling the PPG lamp can further avoid interference caused by the PPG lamp to the user when the user performs short-time sleep, so that sleep quality of the user is improved.

Some supplementary descriptions of the embodiments shown in FIG. 1 to FIG. 3 are as follows.
1. In the embodiment shown in FIG. 4, when the historical sleep data is analyzed to determine the falling-asleep moment range of the user, a date type may be distinguished, and the falling-asleep moment range is analyzed based on an actual date type.

It is considered that in actual life, long-time sleep habits of a user in different time periods may vary greatly. For example, generally, a user needs to go to bed and get up early on workdays. However, on holidays, the user may go to bed and get up later. Therefore, when the embodiment shown in FIG. 4 is performed, dates are classified into types in advance in this embodiment of this application. For example, there may be two time period types: a workday and a holiday (division of the workday and the holiday may be determined based on an actual situation of holiday division in a country in which the user is located, and is not limited herein). Alternatively, there may be five time period types: Monday, Tuesday to Thursday, Friday, Saturday, and Sunday. Then, dates of different time period types are distinguished and analyzed based on specific dates included in an actual past period of time, to obtain an actual available falling-asleep moment range. A specific division rule of the time period types is not limited herein, and may be set by a skilled person based on an actual situation of the user. In addition, the "date" and the "day" in this embodiment of this application may refer to a calendar day, or may refer to a user activity day.

In this embodiment of this application, cases are classified based on occurrence occasions of the embodiment shown in FIG. 4, and different processing solutions are respectively set. Details are described as follows:
For a case in which the embodiment shown in FIG. 4 occurs in a process of S302, in an optional embodiment of this application, logic of S302 is as follows: When it is detected that the user falls asleep, S401 and S402 are performed, to obtain the falling-asleep moment range. Then, it is identified whether the falling-asleep moment of the user is within the falling-asleep moment range.

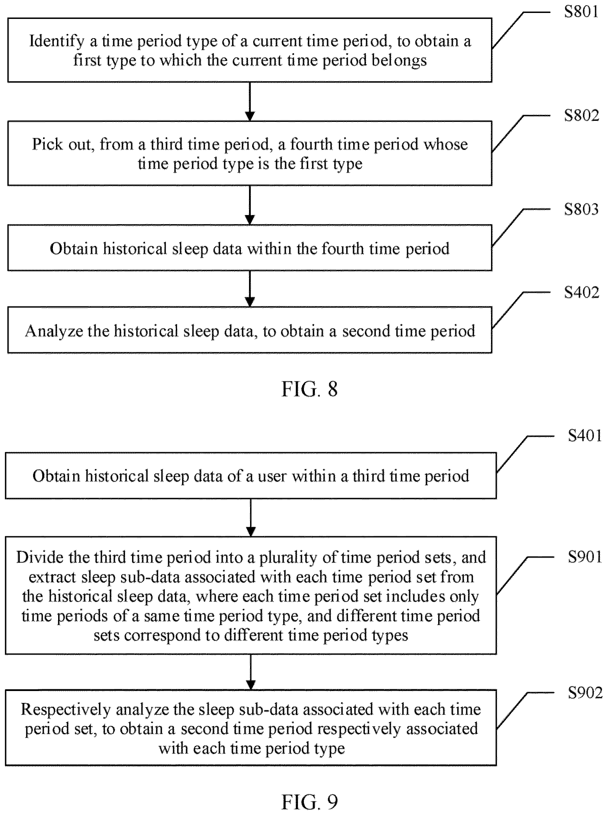

As shown in FIG. 8, in this case, the operation in S401 may be replaced with the following.

S801: Identify a time period type of a current time period, to obtain a first type to which the current time period belongs.

In this embodiment of this application, after historical sleep data of a past period of time is obtained, data analysis is not directly performed, but a time period type (namely, the first type) of the current time period is identified. A unit of the current time period is a minimum time period unit included when the time period types are obtained through division. For example, the minimum time period unit is "day" when holidays, workdays, weekdays, and the like are obtained through division. Therefore, in this case, the current time period refers to a current day. For example, it is identified whether the current day is a workday or a holiday. Alternatively, it is identified that the current day is a specific weekday. When the minimum time period unit is "month", for example, January to March, April to June, July to September, and October to December, the current time period refers to a current month.

S802: Pick out, from the third time period, a fourth time period whose time period type is the first type.

After the time period type of the current time period is identified, all time periods (that is, the fourth time period) belonging to the time period type are picked out from the past period of time. For example, it is assumed that the current day is a holiday. In this case, time periods of all holidays are picked out from the past period of time in this embodiment of this application.

S803: Obtain the historical sleep data within the fourth time period.

After a time period whose type is the same as that of the current time period is picked out, in this embodiment of this application, historical sleep data of these time periods is obtained, and the analysis operation in S402 is performed.

In this embodiment of this application, when a falling-asleep time period is analyzed, a time period of a time period type to which the current time period belongs is selected from a past time period, and historical sleep data in these time periods is obtained in a targeted manner. Therefore, in S402, a falling-asleep moment range can be analyzed based on the time period type to which the current time period belongs, to implement accurate learning and identification for the sleep habit of the user. In this way, in this embodiment of this application, identification for long-time sleep of the user is more accurate, and the sensor is enabled at a more accurate and effective occasion.

The embodiment shown in FIG. 4 is a case occurring after the physiological data during long-time sleep is successfully obtained last time and before S302 (refer to the descriptions of S309 and S310). In another optional embodiment of this application, in this case, it is equivalent to that the falling-asleep moment range is analyzed and stored in advance. When the operation in S302 is performed, the stored falling-asleep moment range is directly read. Correspondingly, as shown in FIG. 9, the operation in S402 may be replaced with the following.

S901: Divide the third time period into a plurality of time period sets, and extract sleep sub-data associated with each time period set from the historical sleep data, where each time period set includes only time periods of a same time period type, and different time period sets correspond to different time period types.

In this embodiment of this application, the past period of time is divided into a plurality of time period sets based on the time period types. The time period sets and the time period types are in a one-to-one correspondence. For example, it is assumed that the time period types include workdays and holidays. In this case, the past period of time is divided into two time period sets in total: a workday set and a holiday set.

After the time period division is completed, in this embodiment of this application, data extraction is performed on the historical sleep data. In other words, historical sleep data corresponding to each time period set is respectively extracted. In this case, each time period set is associated with one piece of sleep sub-data. The sleep sub-data is a part of the historical sleep data, and data formats of the sleep sub-data are the same. A single piece of sleep sub-data includes sleep data of the user per day in a corresponding time period set, for example, a falling-asleep moment per day.

S902: Respectively analyze the sleep sub-data associated with each time period set, to obtain the second time period respectively associated with each time period type.

After the sleep sub-data associated with each time period set is obtained, in this embodiment of this application, each piece of sleep sub-data is respectively analyzed, to obtain a falling-asleep moment range that is in a one-to-one correspondence with each time period set. The time period sets are in a one-to-one correspondence with the time period types. Therefore, in this case, the sleep time range associated with each time period type can be obtained. For example, it is assumed that there are two time period sets in total: a workday set and a holiday set. In this case, in this embodiment of this application, sleep sub-data in the workday set and sleep sub-data in the holiday set are respectively analyzed. In addition, a falling-asleep moment range of the user in a workday and a falling-asleep moment range in a holiday are obtained. For a method for analyzing the sleep sub-data, refer to the method for analyzing the historical sleep data in the embodiment shown in FIG. 5 (in this case, the historical sleep data in the embodiment shown in FIG. 5 is replaced with the sleep sub-data).

Correspondingly, S302 may be replaced with the following: When it is detected that the user falls asleep, the wearable device identifies a time period type (that is, a second type) of a current time period, and reads the second time period associated with the time period type. The wearable device identifies whether a falling-asleep moment is within the read second time period.

On a basis of obtaining the falling-asleep moment range of the user corresponding to each time period type, in S302, when it is identified whether the falling-asleep moment of the user is within the falling-asleep moment range, the falling-asleep moment range associated with the current time period is first read. Then, it is determined, based on the read falling-asleep moment range, whether the falling asleep of the user is to fall into long-time sleep. For descriptions of the current time period, refer to the related description content in S801. Details are not described herein again.

In this embodiment of this application, each time physiological data of the user during long-time sleep is successfully obtained and the historical sleep data is updated, a falling-asleep habit of the user in each time period type is analyzed timely, to obtain a falling-asleep moment range of the user in each time period type and implement adaptive learning for the falling-asleep habits of the user. However, in the embodiment shown in FIG. 3, after it is detected that the user falls asleep, in S302, a falling-asleep moment can be determined only by reading the falling-asleep moment range of the time period type to which the analyzed current time period belongs. Therefore, in this embodiment of this application, adaptive and accurate learning for a sleep habit of the user can be implemented. In this way, in this embodiment of this application, identification for long-time sleep of the user is more accurate, and the sensor is enabled at a more accurate and effective occasion.

2. A use scenario of this embodiment of this application is not limited to sleep monitoring on the user.

The foregoing embodiments shown in FIG. 1 to FIG. 3 are all described by using a user sleep monitoring scenario. In actual application, the embodiments shown in FIG. 1 to FIG. 3 may be applied to any scenario in which sensor enabling control and physiological data collection need to be performed on long-time sleep of the user. In other words, the embodiments may be applied to another scenario other than the sleep monitoring scenario, for example, a scenario of sleep apnea picking and a scenario of monitoring physiological data of a user during sleep.

3. The embodiment shown in FIG. 1 and the embodiment shown in FIG. 3 are used in combination.

It is considered that both cases may be encountered in a process in which the user actually uses the wearable device. In Case 1, the wearable device cannot obtain historical sleep data of long-time sleep of the user. In Case 2, the wearable device can obtain historical sleep data of long-time sleep of the user. Therefore, in actual application, the wearable device may choose to use the embodiment shown in FIG. 1 or the embodiment shown in FIG. 3 based on actual collection for historical sleep data of the user. For example, during a period of time after the user purchases the wearable device, or a period of time after data of the wearable device is cleared, the wearable device does not collect historical sleep data, or an amount of collected historical sleep data is too small. In this case, the embodiment shown in FIG. 1 may be used for processing. However, after the user uses the wearable device for a period of time, the wearable device has collected historical sleep data of the user within the period of time. In this case, the embodiment shown in FIG. 2 may be used for processing.

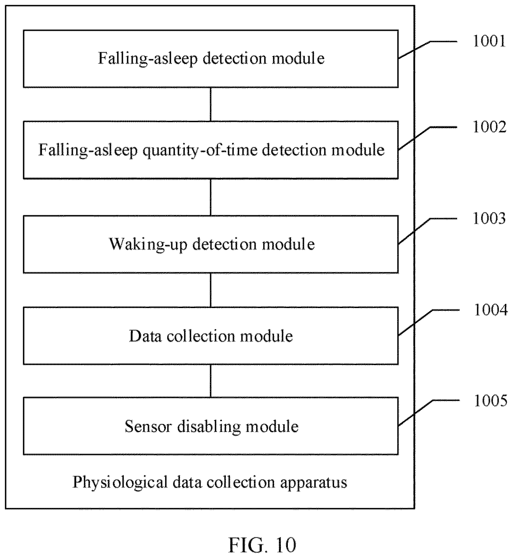

Corresponding to the physiological data collection method in the foregoing embodiment, FIG. 10 shows a block diagram of a structure of a physiological data collection apparatus according to an embodiment of this application. For ease of description, only a part related to this embodiment of this application is shown.

As shown in FIG. 10, the physiological data collection apparatus includes:
a falling-asleep detection module 1001, configured to detect whether a user falls asleep;
a falling-asleep quantity-of-time detection module 1002, configured to: when it is detected that the user falls asleep, identify whether a first quantity of times that the user enters long-time sleep within a first time period is less than a quantity-of-time threshold;
a waking-up detection module 1003, configured to detect whether the user wakes up;
a data collection module 1004, configured to: when a threshold of the first quantity of times is less than the quantity-of-time threshold, enable a first sensor, and collect first physiological data of the user by using the first sensor; and
a sensor disabling module 1005, configured to: when it is detected that the user wakes up, disable the first sensor.

In an optional embodiment of this application, the falling-asleep quantity-of-time detection module 1002 includes:
a falling-asleep moment identification module, configured to: when it is detected that the user falls asleep, identify whether a falling-asleep moment is within a second time period; and
a quantity-of-time detection module, configured to: when the falling-asleep moment is within the second time period, identify whether the first quantity of times that the user enters long-time sleep within the first time period is less than the quantity-of-time threshold.

For details of a process in which the modules in the physiological data collection apparatus provided in this embodiment of this application implement respective functions, refer to the descriptions of the embodiments shown in FIG. 1 to FIG. 3 and other related method embodiments. Details are not described herein again.

It should be noted that content such as information exchange between the foregoing apparatuses/units and execution processes thereof is based on a same concept as the method embodiments of this application For specific functions and technical effects of the content, refer to the method embodiments. Details are not described herein again.

It should be understood that sequence numbers of the steps do not mean an execution sequence in the foregoing embodiments. The execution sequence of the processes should be determined based on functions and internal logic of the processes, and should not constitute any limitation on the implementation processes of embodiments of this application.

It should be understood that, when used in the specification and the appended claims of this application, the term "comprises" indicates existence of the described features, wholes, steps, operations, elements, and/or components. However, the existence or addition of one or more other features, wholes, steps, operations, elements, components, and/or sets thereof is not excluded.

It should be further understood that the term "and/or" used in the specification and the appended claims of this application refers to any combination or all possible combinations of one or more of the items listed in association, and includes these combinations.

As used in the specification and the appended claims of this application, the term "if" may be explained, according to the context, as "when" or "once" or "in response to determining" or "in response to detecting". Similarly, the phrase "if determining" or "if [the described condition or event] is detected" may be interpreted, according to the context, to mean "once determining" or "in response to determining" or "once [the described condition or event] is detected" or "in response to detecting [the described condition or event]."

In addition, in the descriptions of the specification and the appended claims of this application, the terms "first", "second", "third", and the like are merely used for differentiation and description, and shall not be understood as an indication or implication of relative importance. It should be further understood that although the terms "first", "second", and the like are used to describe various elements in some embodiments of this application in the text, these elements should not be limited by these terms. These terms are merely used to distinguish one element from another. For example, a first table may be named a second table, and similarly, the second table may be named the first table, without departing from the scope of the various described embodiments. Both the first table and the second table are tables, but the first table and the second table are not a same table.

Reference to "one embodiment" or "some embodiments" described in this specification of this application means that one or more embodiments of this application include a particular feature, structure, or characteristic described with reference to the embodiments. Therefore, statements such as "in one embodiment", "in some embodiments", "in some other embodiments", and "in still some other embodiments" that appear in this specification and differ from each other do not necessarily refer to a same embodiment; instead, it means "one or more, but not all, embodiments", unless otherwise specifically emphasized. The terms "comprise", "include", "have", and other variants thereof all mean "include but are not limited to", unless specifically emphasized in another manner.

By way of example, rather than limitation, in embodiments of this application, the wearable device may be a generic term for wearable devices developed by intelligently designing daily wear by using a wearable technology, such as glasses, gloves, watches, clothes, and shoes. The wearable device is a portable device that can be directly worn by a user or integrated into clothes or an accessory of a user. The wearable device is more than a hardware device. The wearable device implements powerful functions through software support, data exchange, and cloud interaction. In a broad sense, the wearable smart device has full functions and a large size, and can implement all or partial functions without depending on a smartphone, for example, a smart watch, smart glasses, or the like. In addition, the wearable smart device focuses only on one type of application function, and needs to be used together with another device such as a smartphone, for example, various smart bands or smart jewelries that perform sign monitoring.

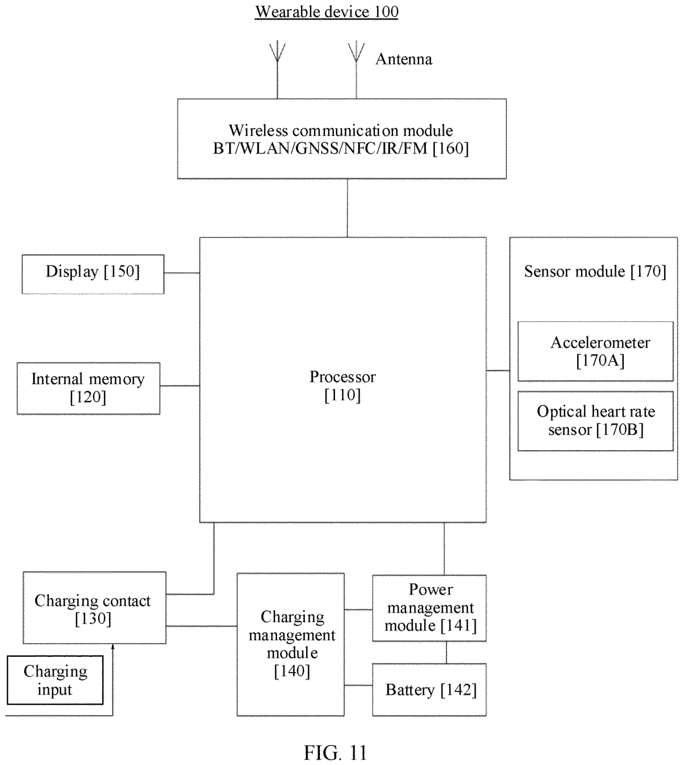

FIG. 11 is a schematic diagram of a structure of a wearable device 100 according to an embodiment of this application.

The wearable device 100 may include a processor 110, an internal memory 120, a charging contact 130, a charging management module 140, a power management module 141, a battery 142, a display 150, an antenna, a wireless communication module 160, a sensor module 170, and the like. The sensor module 170 may include an accelerometer 170A and an optical heart rate sensor 170B. The optical heart rate sensor 170B includes a PPG lamp and a photosensitive sensor.

It may be understood that the structure illustrated in this embodiment of the present invention does not constitute a specific limitation on the wearable device 100. In some other embodiments of this application, the wearable device 100 may include more or fewer components than those shown in the figure, or combine some components, or split some components, or have a different component arrangement. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (Neural-network Processing Unit, NPU), and/or the like. Different processing units may be independent components, or may be integrated into one or more processors. The controller may be a nerve center and a command center of the wearable device 100. The controller may generate an operation control signal based on instruction operation code and a time sequence signal, to control instruction fetching and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor 110 may directly invoke the instructions or the data from the memory. In this way, repeated access is avoided, waiting time of the processor 110 is reduced, and system efficiency is improved.

The processor 110 may run the physiological data collection method provided in embodiments of this application, to implement a low power consumption function for physiological data of a user during long-time sleep, and improve user experience.

The display 150 is configured to display an image, a video, and the like. The display 150 includes a display panel. The display panel may use a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flex light-emitting diode, FLED), a mini LED, a micro LED, a micro OLED, a quantum dot light emitting diode (quantum dot light emitting diodes, QLED), or the like. The display 150 may be configured to display information input by a user or information provided for a user, and various graphical user interfaces (graphical user interface, GUI). For example, the display 150 may display a photo, a video, a web page, a file, or the like. For another example, the display 150 may display a graphical user interface. The graphical user interface includes a status bar, a navigation bar that can be hidden, a time and weather widget (widget), and an application icon, for example, a browser icon. The status bar includes time and a remaining battery level. The navigation bar includes a back (back) button icon, a home (home) button icon, and a forward button icon. In addition, it may be understood that in some embodiments, the status bar may further include a Bluetooth icon, a Wi-Fi icon, an externally-connected device icon, and the like. It may be further understood that, in some other embodiments, the graphical user interface may further include a Dock bar, and the Dock bar may include a commonly used application icon and the like. After detecting a touch event performed by using a finger of the user (or a stylus or the like) on an application icon, the processor opens, in response to the touch event, a user interface of an application corresponding to the application icon, and displays the user interface of the application on the display 150.

In this embodiment of this application, the display 150 may be an integrated flexible display, or may be a spliced display including two rigid screens and one flexible display located between the two rigid screens.

The internal memory 120 may be configured to store computer executable program code, where the executable program code includes instructions. The processor 110 runs the instructions stored in the internal memory 120, to perform various function applications and data processing of the wearable device 100. The internal memory 120 may include a program storage area and a data storage area. The program storage area may store code of an operating system, an application (such as a camera application and a WeChat application), and the like. The data storage area may store data (for example, an image or a video collected by a camera application) created during a process of using the wearable device 100, and the like.

The internal memory 120 may further store one or more computer programs 1200 corresponding to the physiological data collection method provided in embodiments of this application. The one or more computer programs 1200 are stored in the memory 120 and are configured to be executed by the one or more processors 110. The one or more computer programs 1200 include instructions, and the instructions may be used to perform the steps in the corresponding embodiments in FIG. 1 to FIG. 9. When physiological data collection code stored in the internal memory 120 is run by the processor 110, the processor 110 may control the wearable device to perform physiological data collection.

The following describes functions of the sensor module 170.

The accelerometer 170A may collect acceleration data of a wearable device of a user, to determine an action performed by the user after the user wears the wearable device, so as to detect whether the user falls asleep and whether the user wakes up.

The optical heart rate sensor 170B may collect physiological data of the user, to implement user sleep monitoring.

The antenna is configured to transmit and receive electromagnetic wave signals. The wireless communication module 160 may provide a wireless communication solution that is applied to the wearable device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The wireless communication module 160 may be one or more components integrating at least one communication processing module. The wireless communication module 160 receives an electromagnetic wave through an antenna 2, performs frequency modulation and filtering processing on the electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2. In this embodiment of this application, the wireless communication module 160 may be configured to access an access point device, and send and receive a message to and from another wearable device.

It may be understood that an interface connection relationship between modules illustrated in this embodiment of the present invention is merely an example for description, and does not constitute a limitation on a structure of the wearable device 100. In some other embodiments of this application, the wearable device 100 may alternatively use an interface connection manner different from that in the foregoing embodiment, or a combination of a plurality of interface connection manners.

The charging contact is configured to connect to a charger, to charge the wearable device 100. Alternatively, in some optional embodiments, a USB interface may be used to replace the charging contact. The USB interface is an interface that conforms to a USB standard specification, and may be specifically a Mini USB interface, a Micro USB interface, a USB Type-C interface, or the like. The USB interface may be configured to connect to a charger to charge the wearable device 100, or may be configured to transmit data between the wearable device 100 and a peripheral device, or may be configured to connect to a headset, to play audio through the headset. The interface may be further configured to connect to another electronic device such as an AR device.

The charging management module 140 is configured to receive a charging input from a charger. The charger may be a wireless charger or a wired charger. In some wired charging embodiments, the charging management module 140 may receive a charging input from a wired charger through the USB interface 130. In some wireless charging embodiments, the charging management module 140 may receive a wireless charging input through a wireless charging coil of the wearable device 100. When charging the battery 142, the charging management module 140 may further supply power to the electronic device by using the power management module 141.

The power management module 141 is configured to connect to the battery 142, the charging management module 140, and the processor 110. The power management module 141 receives an input from the battery 142 and/or the charging management module 140, and supplies power to the processor 110, the internal memory 120, the display 150, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same component.

It should be understood that, in actual application, the wearable device 100 may include more or fewer components than those shown in FIG. 1. This is not limited in this embodiment of this application. The wearable device 100 shown in the figure is merely an example. The wearable device 100 may have more or fewer components than those shown in the figure, may combine two or more components, or may have different component configurations. The various components shown in the figure may be implemented in hardware including one or more signal processing and/or application-specific integrated circuits, software, or a combination of hardware and software.

In addition, functional units in embodiments of this application may be integrated into one processing unit, or each of the units may exist alone physically, or two or more units may be integrated into one unit. The integrated unit may be implemented in a form of hardware, or may be implemented in a form of a software functional unit.

An embodiment of this application further provides a computer-readable storage medium. The computer-readable storage medium stores a computer program, and when the computer program is executed by a processor, the steps in the foregoing method embodiments can be implemented.

An embodiment of this application further provides a computer program product. When the computer program product runs on a wearable device, the wearable device is enabled to implement the steps in the foregoing method embodiments.

An embodiment of this application further provides a chip system. The chip system includes a processor, and the processor is coupled to a memory. The processor executes a computer program stored in the memory, to implement the steps in the foregoing method embodiments.

When the integrated module/unit is implemented in a form of a software functional unit and sold or used as an independent product, the integrated module/unit may be stored in a computer-readable storage medium. Based on such an understanding, in this application, all or some of the processes in the methods in the foregoing embodiments may alternatively be implemented by using a computer program to instruct related hardware. The computer program may be stored in a computer-readable storage medium. When the computer program is executed by a processor, the steps in the foregoing method embodiments may be implemented. The computer program includes computer program code, and the computer program code may be in a source code form, an object code form, an executable file form, some intermediate forms, or the like. The computer-readable storage medium may include any entity or apparatus that can carry the computer program code, a recording medium, a USB flash drive, a removable hard disk, a magnetic disk, an optical disk, a computer memory, a read-only memory (Read-Only Memory, ROM), a random access memory (Random Access Memory, RAM), an electrical carrier signal, a telecommunication signal, a software distribution medium, and the like.

In the foregoing embodiments, descriptions for all embodiments have respective focuses. For a part that is not described in detail or recorded in an embodiment, refer to the related descriptions in another embodiment.

A person of ordinary skill in the art may be aware that units and algorithm steps in the examples described with reference to embodiments disclosed in this specification may be implemented by electronic hardware or a combination of computer software and electronic hardware. Whether the functions are executed by hardware or software depends on a particular application and a design constraint condition that are of the technical solutions. A person skilled in the art may use different methods to implement the described functions for each particular application.

The units described as separate parts may or may not be physically separate, and parts displayed as units may or may not be physical units, that is, may be located in one location, or may be distributed on a plurality of network units. Some or all of the units may be selected based on an actual requirement to achieve the objectives of the solutions of the embodiments.

The foregoing embodiments are merely used to describe the technical solutions of this application, but are not intended to limit this application. Although this application is described in detail with reference to the foregoing embodiments, a person of ordinary skill in the art should understand that the technical solutions described in the foregoing embodiments may still be modified, or some technical features thereof may be equivalently replaced.

Finally, it should be noted that the foregoing descriptions are merely specific implementations of this application, but are not intended to limit the protection scope of this application. The current invention is defined by the scope of the claims.

## Claims

1. A physiological data collection method, comprising:
detecting whether a user falls asleep;
identifying, when it is detected that the user falls asleep, whether a first quantity of times that the user enters long-time sleep within a first time period is less than a quantity-of-time threshold, and detecting whether the user wakes up;
when the first quantity of times is less than the quantity-of-time threshold, enabling a first sensor, and collecting first physiological data of the user by using the first sensor; and
when it is detected that the user wakes up, disabling the first sensor.

2. The physiological data collection method according to claim 1, wherein the identifying, when it is detected that the user falls asleep, whether a first quantity of times that the user enters long-time sleep within a first time period is less than a quantity-of-time threshold comprises:
identifying, when it is detected that the user falls asleep, whether a falling-asleep moment is within a second time period, wherein the second time period is the period during which the user enters long-time sleep; and
when the falling-asleep moment is within the second time period, identifying whether the first quantity of times that the user enters long-time sleep within the first time period is less than the quantity-of-time threshold.

3. The physiological data collection method according to claim 2, wherein before the identifying whether a falling-asleep moment is within a second time period, the method further comprises:
obtaining historical sleep data of the user within a third time period; and
analyzing the historical sleep data to obtain the second time period.

4. The physiological data collection method according to claim 3, wherein the analyzing the historical sleep data to obtain the second time period comprises:
analyzing the historical sleep data, to obtain sleep period data that is associated with k times of sleep in which the user sleeps for the longest sleep duration each day and that are within the third time period, wherein k is equal to the quantity-of-time threshold;
picking out, from the sleep period data, sleep period data that is associated with a sleep period whose sleep duration is greater than or equal to a duration threshold, and reading a falling-asleep moment comprised in each piece of picked-out sleep period data; and
analyzing the read falling-asleep moment, to obtain the second time period.

5. The physiological data collection method according to any one of claims 1 to 4, further comprising:
when it is detected that the user wakes up, collecting statistics on first duration lasting from the moment at which the user falls asleep to the moment at which the user wakes up; and
when the first duration is greater than or equal to the duration threshold, determining that the sleep is long-time sleep, and updating the first quantity of times.

6. The physiological data collection method according to any one of claims 1 to 4, further comprising:
when it is detected that the user wakes up, collecting statistics on first duration lasting from the moment at which the user falls asleep to the moment at which the user wakes up;
when the first duration is less than the duration threshold, detecting whether the user falls asleep;
when it is detected that the user falls asleep, detecting whether the user wakes up, enabling the first sensor, and collecting second physiological data of the user by using the first sensor.

7. The physiological data collection method according to claim 3 or 4, wherein the obtaining historical sleep data of the user within a third time period comprising:
identifying a time period type of a current time period;
picking out, from the third time period, a fourth time period whose time period type is a first type; and
obtaining the historical sleep data within the fourth time period.

8. The physiological data collection method according to claim 3 or 4, wherein the analyzing the historical sleep data, to obtain the second time period comprises:
dividing the third time period into a plurality of time period sets, and extracting sleep sub-data of each time period set from the historical sleep data, wherein each time period set comprises only time periods of a same time period type, and different time period sets are associated with different time period types; and
respectively analyzing the sleep sub-data associated with each time period set, to obtain the second time period respectively associated with each time period type; and
correspondingly, the identifying, when it is detected that the user falls asleep, whether a falling-asleep moment is within a second time period comprises:
when it is detected that the user falls asleep, identifying a time period type of a current time period, to obtain a second type; and
obtaining the second time period associated with the second type, and identifying whether the falling-asleep moment is within the second time period.

9. The physiological data collection method according to claim 1, wherein the identifying, when it is detected that the user falls asleep, whether a first quantity of times that the user enters long-time sleep within a first time period is less than a quantity-of-time threshold comprises:
when it is detected that the user falls asleep, identifying whether the sleep is the first time that the user falls asleep within the first time period; and
when the sleep is the first time that the user falls asleep within the first time period, determining that the threshold of the first quantity of times is less than the quantity-of-time threshold.

10. The physiological data collection method according to claim 1, wherein the first sensor is a PPG sensor.

11. A electronic device, wherein the electronic device comprises a memory, a processor, and a first sensor, the memory stores a computer program that can be run on the processor, and when the processor executes the computer program, steps of the method according to any one of claims 1 to 10 are implemented.

12. The electronic device according to claim 11, wherein the electronic device is a wearable device.

13. A computer-readable storage medium, wherein the computer-readable storage medium stores a computer program, and when the computer program is executed by a processor, steps of the method according to any one of claims 1 to 10 are implemented.

14. A chip system, wherein the chip system comprises a processor, the processor is coupled to a memory, and the processor executes a computer program stored in the memory, to implement the physiological data collection method according to any one of claims 1 to 10.

## Patentansprüche

1. Verfahren zur Erfassung physiologischer Daten, umfassend:
Erkennen, ob ein Benutzer einschläft;
wenn erkannt wird, dass der Benutzer einschläft, Identifizieren, ob eine erste Häufigkeit, mit der der Benutzer innerhalb eines ersten Zeitraums in einen Langzeitschlaf eintritt, kleiner ist als ein Zeitmengenschwellenwert, und Erkennen, ob der Benutzer aufwacht;
wenn die erste Zeitmenge kleiner als der Zeitmengenschwellenwert ist, Aktivieren eines ersten Sensors und Erfassen erster physiologischer Daten des Benutzers unter Verwendung des ersten Sensors; und
wenn erkannt wird, dass der Benutzer aufwacht, Deaktivieren des ersten Sensors.

2. Verfahren zur Erfassung physiologischer Daten nach Anspruch 1, wobei das Identifizieren, wenn erkannt wird, dass der Benutzer einschläft, ob eine erste Häufigkeit, mit denen der Benutzer innerhalb eines ersten Zeitraums in Langzeitschlaf eintritt, kleiner als ein Zeitmengenschwellenwert ist, Folgendes umfasst:
Identifizieren, wenn erkannt wird, dass der Benutzer einschläft, ob ein Einschlafmoment innerhalb eines zweiten Zeitraums liegt, wobei der zweite Zeitraum der Zeitraum ist, in dem der Benutzer in einen Langzeitschlaf eintritt; und
wenn der Einschlafmoment innerhalb des zweiten Zeitraums liegt, Identifizieren, ob die erste Häufigkeit, die der Benutzer innerhalb des ersten Zeitraums in einen Langzeitschlaf eintritt, kleiner als der Zeitmengenschwellenwert ist.

3. Verfahren zur Erfassung physiologischer Daten nach Anspruch 2, wobei das Verfahren vor dem Identifizieren, ob ein Einschlafmoment innerhalb eines zweiten Zeitraums liegt, ferner Folgendes umfasst:
Erhalten historischer Schlafdaten des Benutzers innerhalb eines dritten Zeitraums; und
Analysieren der historischen Schlafdaten, um den zweiten Zeitraum zu erhalten.

4. Verfahren zur Erfassung physiologischer Daten nach Anspruch 3, wobei das Analysieren der historischen Schlafdaten zum Erhalten des zweiten Zeitraums Folgendes umfasst:
Analysieren der historischen Schlafdaten, um Schlafperiodendaten zu erhalten, die k Schlafzeiten zugeordnet sind, in denen der Benutzer pro Tag am längsten schläft und die innerhalb des dritten Zeitraums liegen, wobei k gleich dem Zeitmengenschwellenwert ist;
Auswählen von Schlafperiodendaten aus den Schlafperiodendaten, die einer Schlafperiode zugeordnet sind, deren Schlafdauer größer oder gleich einem Dauerschwellenwert ist, und Lesen eines Einschlafmoments, das in jedem Teil der herausgegriffenen Schlafperiodendaten enthalten ist; und
Analysieren des abgelesenen Einschlafmoments, um den zweite Zeitraum zu erhalten.

5. Verfahren zur Erfassung physiologischer Daten nach einem der Ansprüche 1 bis 4, ferner umfassend:
wenn erkannt wird, dass der Benutzer aufwacht, Sammeln von Statistiken über die Dauer vom Einschlafen bis zum Aufwachen des Benutzers; und
wenn die erste Dauer größer oder gleich dem Dauerschwellenwert ist, Bestimmen, dass es sich bei dem Schlaf um Langzeitschlaf handelt, und Aktualisieren der ersten Häufigkeit.

6. Verfahren zur Erfassung physiologischer Daten nach einem der Ansprüche 1 bis 4, ferner umfassend:
wenn erkannt wird, dass der Benutzer aufwacht, Sammeln von Statistiken über die Dauer vom Einschlafen bis zum Aufwachen des Benutzers;
wenn die erste Dauer kürzer als der Dauerschwellenwert ist, Erkennen, ob der Benutzer einschläft;
wenn erkannt wird, dass der Benutzer einschläft, Erkennen, ob der Benutzer aufwacht, Aktivieren des ersten Sensors und Erfassen zweiter physiologischer Daten des Benutzers unter Verwendung des ersten Sensors.

7. Verfahren zum Erfassen physiologischer Daten nach Anspruch 3 oder 4, wobei das Erfassen historischer Schlafdaten des Benutzers innerhalb eines dritten Zeitraums Folgendes umfasst:
Identifizieren eines Zeitraumtyps eines aktuellen Zeitraums;
Auswählen eines vierten Zeitraums aus dem dritten Zeitraum, dessen Zeitraumtyp ein erster Typ ist; und
Abrufen der historischen Schlafdaten innerhalb des vierten Zeitraums.

8. Verfahren zur Erfassung physiologischer Daten nach Anspruch 3 oder 4, wobei das Analysieren der historischen Schlafdaten zum Erhalten des zweiten Zeitraums Folgendes umfasst:
Aufteilen des dritten Zeitraums in eine Vielzahl von Zeitraumsätzen und Extrahieren von Schlafunterdaten jedes Zeitraumsatzes aus den historischen Schlafdaten, wobei jeder Zeitraumsatz nur Zeiträume desselben Zeitraumtyps umfasst und unterschiedliche Zeitraumsätze unterschiedlichen Zeitraumtypen zugeordnet sind; und
jeweiliges Analysieren der mit jedem eingestellten Zeitraum verknüpften Schlafunterdaten, um den jeweils mit jedem Zeitraumtyp zugeordneten zweiten Zeitraum zu erhalten; und
dementsprechend umfasst das Erkennen, wenn erkannt wird, dass der Benutzer einschläft, ob ein Einschlafmoment innerhalb eines zweiten Zeitraums liegt:
wenn erkannt wird, dass der Benutzer einschläft, Identifizieren eines Zeitraumtyps eines aktuellen Zeitraums, um einen zweiten Typ zu erhalten; und
Erhalten des zweiten Zeitraums, der dem zweiten Typ zugeordnet ist, und Identifizieren, ob der Einschlafmoment innerhalb des zweiten Zeitraums liegt.

9. Verfahren zur Erfassung physiologischer Daten nach Anspruch 1, wobei das Identifizieren, wenn erkannt wird, dass der Benutzer einschläft, ob eine erste Häufigkeit, mit denen der Benutzer innerhalb eines ersten Zeitraums in Langzeitschlaf eintritt, kleiner als ein Zeitmengenschwellenwert ist, Folgendes umfasst:
wenn erkannt wird, dass der Benutzer einschläft, Identifizieren, ob der Schlaf das erste Mal ist, dass der Benutzer innerhalb des ersten Zeitraums einschläft; und
wenn der Schlaf das erste Mal ist, dass der Benutzer innerhalb des ersten Zeitraums einschläft, Bestimmen, dass der Schwellenwert der ersten Häufigkeit kleiner als der Zeitmengenschwellenwert ist.

10. Verfahren zur Erfassung physiologischer Daten nach Anspruch 1, wobei der erste Sensor ein PPG-Sensor ist.

11. Elektronische Vorrichtung, wobei die elektronische Vorrichtung einen Speicher, einen Prozessor und einen ersten Sensor umfasst, der Speicher ein Computerprogramm speichert, das auf dem Prozessor ausgeführt werden kann, und wenn der Prozessor das Computerprogramm ausführt, Schritte des Verfahrens nach einem der Ansprüche 1 bis 10 umgesetzt werden.

12. Elektronische Vorrichtung nach Anspruch 11, wobei die elektronische Vorrichtung eine Wearable-Vorrichtung ist.

13. Computerlesbares Speichermedium, wobei das computerlesbare Speichermedium ein Computerprogramm speichert und wenn das Computerprogramm von einem Prozessor ausgeführt wird, Schritte des Verfahrens nach einem der Ansprüche 1 bis 10 umgesetzt werden.

14. Chipsystem, wobei das Chipsystem einen Prozessor umfasst, der Prozessor mit einem Speicher gekoppelt ist und der Prozessor ein im Speicher gespeichertes Computerprogramm ausführt, um das Verfahren zur Erfassung physiologischer Daten gemäß einem der Ansprüche 1 bis 10 umzusetzen.

## Revendications

1. Procédé de collecte de données physiologiques, comprenant :
le fait de détecter si un utilisateur s'endort ;
le fait d'identifier, lorsqu'il est détecté que l'utilisateur s'endort, si une première quantité de fois où l'utilisateur entre en sommeil prolongé au cours d'une première période de temps est inférieure à un seuil de quantité de temps, et le fait de détecter si l'utilisateur se réveille ;
lorsque la première quantité de fois est inférieure au seuil de quantité de temps, l'activation d'un premier capteur et la collecte des premières données physiologiques de l'utilisateur à l'aide du premier capteur ; et
lorsqu'il est détecté que l'utilisateur se réveille, la désactivation du premier capteur.

2. Procédé de collecte de données physiologiques selon la revendication 1, dans lequel le fait d'identifier, lorsqu'il est détecté que l'utilisateur s'endort, si une première quantité de fois où l'utilisateur entre en sommeil prolongé au cours d'une première période de temps est inférieure à un seuil de quantité de temps comprend :
le fait d'identifier, lorsqu'il est détecté que l'utilisateur s'endort, si un moment d'endormissement se situe dans une deuxième période de temps, dans lequel la deuxième période de temps est la période pendant laquelle l'utilisateur entre en sommeil prolongé ; et
lorsque le moment d'endormissement se situe dans la deuxième période de temps, le fait d'identifier si la première quantité de fois où l'utilisateur entre en sommeil prolongé au cours de la première période de temps est inférieure au seuil de quantité de temps.

3. Procédé de collecte de données physiologiques selon la revendication 2, dans lequel avant d'identifier si un moment d'endormissement se situe dans une deuxième période de temps, le procédé comprend également :
l'obtention de données historiques sur le sommeil de l'utilisateur au cours d'une troisième période de temps ; et
l'analyse des données historiques de sommeil pour obtenir la deuxième période de temps.

4. Procédé de collecte de données physiologiques selon la revendication 3, dans lequel l'analyse des données historiques de sommeil pour obtenir la deuxième période de temps comprend :
l'analyse des données historiques de sommeil, pour obtenir des données de période de sommeil qui sont associées à k périodes de sommeil pendant lesquelles l'utilisateur dort pendant la durée de sommeil la plus longue chaque jour et qui se situent dans la troisième période de temps, dans lequel k est égal au seuil de quantité de temps ;
la sélection, à partir des données de période de sommeil, des données de période de sommeil qui sont associées à une période de sommeil dont la durée de sommeil est supérieure ou égale à un seuil de durée, et la lecture d'un moment d'endormissement compris dans chaque élément de données de période de sommeil sélectionnées ; et
l'analyse du moment d'endormissement lu, pour obtenir la deuxième période de temps.

5. Procédé de collecte de données physiologiques selon l'une quelconque des revendications 1 à 4, comprenant également :
lorsqu'il est détecté que l'utilisateur se réveille, la collecte de statistiques sur une première durée allant du moment où l'utilisateur s'endort jusqu'au moment où l'utilisateur se réveille ; et
lorsque la première durée est supérieure ou égale au seuil de durée, la détermination que le sommeil est un sommeil prolongé, et la mise à jour de la première quantité de fois.

6. Procédé de collecte de données physiologiques selon l'une quelconque des revendications 1 à 4, comprenant également :
lorsqu'il est détecté que l'utilisateur se réveille, la collecte de statistiques sur une première durée allant du moment où l'utilisateur s'endort jusqu'au moment où l'utilisateur se réveille ;
lorsque la première durée est inférieure au seuil de durée, le fait de détecter si l'utilisateur s'endort ;
lorsqu'il est détecté que l'utilisateur s'endort, le fait de détecter si l'utilisateur se réveille, l'activation du premier capteur et la collecte de secondes données physiologiques de l'utilisateur à l'aide du premier capteur.

7. Procédé de collecte de données physiologiques selon la revendication 3 ou 4, dans lequel l'obtention de données historiques sur le sommeil de l'utilisateur au cours d'une troisième période de temps comprend :
l'identification d'un type de période de temps d'une période de temps actuelle ;
la sélection, à partir de la troisième période de temps, d'une quatrième période de temps dont le type de période de temps est un premier type ; et
l'obtention des données historiques de sommeil au cours de la quatrième période de temps.

8. Procédé de collecte de données physiologiques selon la revendication 3 ou 4, dans lequel l'analyse des données historiques de sommeil pour obtenir la deuxième période de temps comprend :
la division de la troisième période de temps en une pluralité d'ensembles de périodes de temps, et l'extraction des sous-données de sommeil de chaque ensemble de périodes de temps à partir des données historiques de sommeil, dans lequel chaque ensemble de périodes de temps comprend uniquement des périodes de temps d'un même type de période de temps, et différents ensembles de périodes de temps sont associés à différents types de périodes de temps ; et
l'analyse respective des sous-données de sommeil associées à chaque ensemble de périodes de temps, pour obtenir la deuxième période de temps respectivement associée à chaque type de période de temps ; et
de manière correspondante, le fait d'identifier, lorsqu'il est détecté que l'utilisateur s'endort, si un moment d'endormissement se situe dans une deuxième période de temps comprend :
lorsqu'il est détecté que l'utilisateur s'endort, le fait d'identifier un type de période de temps d'une période de temps actuelle, pour obtenir un second type ; et
l'obtention de la deuxième période de temps associée au second type, et le fait identifier si le moment d'endormissement se situe dans la deuxième période de temps.

9. Procédé de collecte de données physiologiques selon la revendication 1, dans lequel le fait d'identifier, lorsqu'il est détecté que l'utilisateur s'endort, si une première quantité de fois où l'utilisateur entre en sommeil prolongé au cours d'une première période de temps est inférieure à un seuil de quantité de temps comprend :
lorsqu'il est détecté que l'utilisateur s'endort, le fait d'identifier si le sommeil est la première fois que l'utilisateur s'endort au cours de la première période de temps ; et
lorsque le sommeil est la première fois que l'utilisateur s'endort au cours de la première période de temps, la détermination que le seuil de la première quantité de fois est inférieur au seuil de quantité de temps.

10. Procédé de collecte de données physiologiques selon la revendication 1, dans lequel le premier capteur est un capteur PPG.

11. Dispositif électronique, dans lequel le dispositif électronique comprend une mémoire, un processeur et un premier capteur, la mémoire stocke un programme informatique qui peut être exécuté par le processeur, et lorsque le processeur exécute le programme informatique, les étapes du procédé selon l'une quelconque des revendications 1 à 10 sont mises en oeuvre.

12. Dispositif électronique selon la revendication 11, dans lequel le dispositif électronique est un dispositif à porter sur soi.

13. Support de stockage lisible par ordinateur, dans lequel le support de stockage lisible par ordinateur stocke un programme informatique, et lorsque le programme informatique est exécuté par un processeur, les étapes du procédé selon l'une quelconque des revendications 1 à 10 sont mises en oeuvre.

14. Système à puce, dans lequel le système à puce comprend un processeur, le processeur est couplé à une mémoire, et le processeur exécute un programme informatique stocké dans la mémoire, pour mettre en oeuvre le procédé de collecte de données physiologiques selon l'une quelconque des revendications 1 à 10.
